# EUROPEAN PATENT SPECIFICATION

(11) **EP 1 667 947 B1**
(45) Date of publication and mention of the grant of the patent: **20.08.2008**
(21) Application number: 04768696.9
(22) Date of filing: 27.09.2004
(51) Int. Cl.: C07B 59/00, C07D 279/20, A61K 31/5415, A61P 35/00

(54) **METHODS OF 11C -RADIOLABELLING PHENOTHIAZINE AND PHENO THIAZINE-LIKE COMPOUNDS**
VERFAHREN ZUR [11C]-RADIOMARKIERUNG VON PHENTHIAZIN UND PHENOTHIAZINARTIGE VERBINDUNGEN
METHODES DE MARQUAGE 11C DE LA PHENOTHIAZINE ET DE COMPOSES SEMBLABLES A LA PHENOTHIAZINE

(30) Priority: 29.09.2003 GB 0322756
(43) Date of publication of application: 14.06.2006
(73) Proprietor: The University Court of the University of Aberdeen, Aberdeenshire AB24 3FX (GB)
(72) Inventor: SCHWEIGER, Lutz F., c/o University of Aberdeen, Aberdeen, Aberdeenshire AB25 2ZD (GB); CRAIB, Stuart, A., c/o University of Aberdeen, Aberdeen, Aberdeenshire AB25 2ZD (GB); WELCH, Andrew, E., c/o University of Aberdeen, Aberdeen, Aberdeenshire AB25 2ZD (GB); SHARP, Peter, F., c/o University of Aberdeen, Aberdeen, Aberdeenshire AB25 2ZD (GB)
(74) Representative: Wytenburg, Wilhelmus Johannes
(86) International application number: PCT/GB2004/004153
(87) International publication number: WO 2005/030676

(56) References cited:
- TURNER J ET AL.: "Localization of carbon-11 radiopharmaceuticals in the Greene melanoma of hamsters" EUR. J. NUCL. MED., vol. 10, no. 9-10, 1985, pages 392-397, XP008041008
- R. IWATA ET AL.: "A combined loop-SPE method for the automated preparation of [11C]doxepin" J. LABEL. COMP. RADIOPHARM., vol. 45, 2002, pages 271-280, XP002312361
- LUTZ SCHWEIGER ET AL.: "Radiosynthesis of [N-methyl-11C]methylene blue" J. LABEL. COMP. RADIOPHARM., vol. 46, November 2003 (2003-11), pages 1221-1228, XP002312362

## Description

### TECHNICAL FIELD

This invention pertains generally to the field of radiochemical synthesis, and more specifically to methods of [¹¹C]-radiolabelling "phenothiazine" and "phenothiazine-like" compounds, which have a pendant group (which is a primary amino group; a cationic primary imino group; a secondary amino group; a cationic secondary imino group; a primary imino group; or a secondary imino group), by reaction with [¹¹C]methyl trifluoromethanesulfonate (CF₃SO₂O¹¹CH₃), also known as [¹¹C]methyl triflate. This reaction converts the pendant group into a [¹¹C]methyl-labelled pendant group. The resulting [¹¹C]-radiolabelling product is useful, for example, as an in vivo positron emission tomography (PET) tracer, for example, for patients suffering from melanoma, the most serious form of skin cancer, and tauopathy (e.g., Alzheimer's disease). The present invention also pertains to the resulting [¹¹C]-radiolabelling products, compositions comprising them, their use in methods of (e.g., PET) imaging, their use in methods of medical treatment and diagnosis, etc.

### BACKGROUND

Throughout this specification, including any claims which follow, unless the context requires otherwise, the word "comprise," and variations such as "comprises" and "comprising," will be understood to imply the inclusion of a stated integer or step or group of integers or steps, but not the exclusion of any other integer or step or group of integers or steps.

It must be noted that, as used in the specification and any appended claims, the singular forms "a," "an," and "the" include plural referents unless the context clearly dictates otherwise. Thus, for example, reference to "a pharmaceutical carrier" includes mixtures of two or more such carriers, and the like.

Ranges are often expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

### Melanoma

Melanoma is the most serious form of skin cancer and claims around 2,000 lives each year in the United Kingdom of Great Britain (see, e.g., Cancer Research UK Website).

According to Cancer Research UK (see, e.g., Cancer Research UK Website) malignant melanoma is the 11th most common cancer in women, and the 12th most common cancer in men with over 5,700 new cases of melanoma each year in the UK.

Melanoma develops from cells producing melanin, a pigment that protects the deeper layers of the skin from the damaging effects of the sun:

### Methylene Blue

Methylene blue (3,7-bis(dimethylamino)phenothiazine-5-ium chloride) is a low molecular weight, water soluble, tricyclic organic compound, which diffuses through the cellular membranes and accumulates selectively in melanoma cells (see, e.g., Link et a., 1998).

Methylene blue possesses a very high affinity to melanin by forming a charge transfer complex with the pigment (see, e.g., Potts, 1964).

Over several years, Link et al. have carried out clinical research focusing on methylene blue labelled with relatively long lived radioisotopes such as ²¹¹Astatine (²¹¹At, half-life (t_{½}) = 7.2 hours), ¹²³Iodine (¹²¹I, t_{½} = 13.2 hours) and ¹³¹Iodine (¹³¹I, t_{½} = 8 days) (see, e.g., Link et al., 1998).

They investigated the α-particle emitter compound [²¹¹At]methylene blue as a therapeutic agent and were able to prove that this radioactive compound prevents metastatic spread and controls the growth of melanoma when given to human-melanoma-bearing animals (see, e.g., Link et al., 1998). They also investigated the γ-emitting ¹²³I- and the β-emitting [¹³¹I]methylene blue compounds for diagnostic purposes of disseminated melanoma. Using a gamma camera, they concluded that in particular the ¹³¹I labelled compound was suitable for the detection of melanoma metastases (see, e.g., Link et al., 1998).

There is a great need for additional, and more powerful, radiolabelled phenothiazine and phenothiazine-like compounds, such as methylene blue.

The inventors have discovered novel methods for the fast and efficient synthesis of novel phenothiazine and phenothiazine-like compounds labelled with the short lived positron emitting ¹¹C isotope (t_{1/2}= 20.4 minutes).

It is surprising and unexpected that the synthesis method is both fast (e.g., fast enough to compensate for the short half life), and efficient (e.g., efficient enough to provide sufficient radioactive yield to be useful).

¹¹C-labelled methylene blue is structurally identical to unlabelled methylene blue, and hence would show the same biodistribution, which is important for PET studies. Therefore [N-methyl-¹¹C]methylene blue is very useful, in particular as an in vivo PET tracer for patients suffering from melanoma, the most serious form of skin cancer, tauopathy (e.g., Alzheimer's disease), and other diseases.

Turner et al., Eur. J. Nucl. Med., 1985, Vol. 10, pp. 392-397 describes a method for preparing ¹¹C-chlorpromazine using the monodesmethyl derivative of chlorpromazine and ¹¹C-formaldehyde (prepared by dehydrogenation of ¹¹C-methanol). See, for example, "Materials and Methods" on page 392 therein. 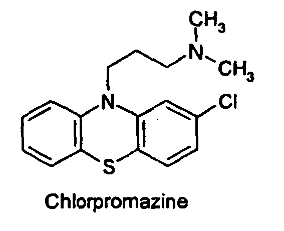 Iwata et al., J. Label. Compd. Radiopharm., 2002, Vol. 45, pp. 271-280 describes a combined loop-solid phase extraction (SPE) method for the automated preparation of [¹¹C]-doxepin from nordoxepin using [¹¹C]methyl triflate ([¹¹C]MeOTf). See, for example, Scheme 1 on page 275 therein. 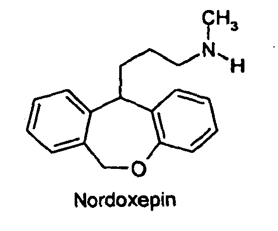 One aspect of the present invention pertains to a method of [¹¹C]-radiolabelling a phenothiazine compound or a phenothiazine-like compound, wherein:
said compound has a polycyclic core of three six-membered rings fused together in a linear fashion and denoted the A-ring, B-ring, and C-ring, where the B-ring is the "middle" ring;
said polycyclic core is partially-aromatic or fully-aromatic;
said polycyclic core has 14 ring atoms, including exactly 1 or exactly 2 ring heteroatom(s), each of which is independently selected from N, O, and S;
the remainder of said ring atoms being C;
said exactly 1 or exactly 2 ring heteroatom(s) form part of the B-ring, but not part of the A-ring or C-ring, and so are located at one or both of the "central" positions denoted by a hash-mark (#) in the following depiction of the polycyclic core: 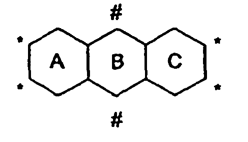
said compound has a pendant group covalently attached to a ring atom of said polycyclic core;
said pendant group is independently:
   a primary amino group;
   a cationic primary imino group;
   a secondary amino group;
   a cationic secondary imino group;
   a primary imino group; or
   a secondary imino group;
said method comprising the step of:
   reacting said phenothiazine compound or a phenothiazine-like compound with [¹¹C]methyl trifluoromethanesulfonate (CF₃SO₂O¹¹CH₃);
   thereby converting said pendant group to a corresponding [¹¹C]methyl-labelled pendant group, respectively:
      a [¹¹C]methyl-labelled secondary amino group;
      a [¹¹C]methyl-labelled cationic secondary imino group;
      a [¹¹C]methyl-labelled tertiary amino group;
      a [¹¹C]methyl-labelled cationic tertiary imino group;
      a [¹¹C]methyl-labelled secondary imino group; or
      a [¹¹C]methyl-labelled cationic tertiary imino group;
   to give a [¹¹C]-radiolabelled phenothiazine or phenothiazine-like compound.

Also described herein is a [¹¹C]-radiolabelled phenothiazine or phenothiazine-like compound.

Also described herein is a [¹¹C]-radiolabelled phenothiazine or phenothiazine-like compound which is *obtained by, or obtainable by,* a method as described herein.

Also described herein is a composition (e.g., a pharmaceutical composition) comprising a [¹¹C]-radiolabelled phenothiazine or phenothiazine-like compound as described herein.

Also described herein is a method of PET imaging which employs a [¹¹C]-radiolabelled phenothiazine or phenothiazine-like compound as described herein.

Also described herein is a [¹¹C]-radiolabelled phenothiazine or phenothiazine-like compound as described herein for use in a method of treatment of the human or animal body by therapy.

Also described herein is use of a [¹¹C]-radiolabelled phenothiazine or phenothiazine-like compound as described herein in the manufacture of a medicament for use in the treatment of, e.g., skin cancer (e.g., melanoma) or a tauopathy (e.g., Alzheimer's disease).

Also described herein is a method of [¹¹C]-radiolabelling a phenothiazine compound or a phenothiazine-like compound, as described herein, as part of a method of manufacturing a medicament for use in the treatment of, e.g., skin cancer (e.g., melanoma) a tauopathy (e.g., Alzheimer's disease).

Another aspect of the invention pertains to use of:
(i) an unlabelled phenothiazine compound or an unlabelled phenothiazine-like compound, wherein:
   said compound has a polycyclic core of three six-membered rings fused together in a linear fashion and denoted the A-ring, B-ring, and C-ring, where the B-ring is the "middle" ring;
   said polycyclic core is partially-aromatic or fully-aromatic;
   said polycyclic core has 14 ring atoms, including exactly 1 or exactly 2 ring heteroatom(s), each of which is independently selected from N, O, and S;
   the remainder of said ring atoms being C;
   said exactly 1 or exactly 2 ring heteroatom(s) form part of the B-ring, but not part of the A-ring or C-ring, and so are located at one or both of the "central" positions denoted by a hash-mark (#) in the following depiction of the polycyclic core: 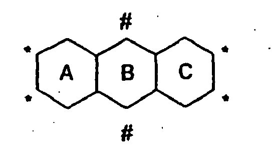
   said compound has a pendant group covalently attached to a ring atom of said polycyclic core;
   said pendant group is independently:
      a primary amino group;
      a cationic primary imino group;
      a secondary amino group;
      a cationic secondary imino group;
      a primary imino group; or
      a secondary imino group;
   and
(ii) [¹¹C]methyl trifluoromethanesulfonate (CF₃SO₂O¹¹CH₃);
   in the manufacture of a medicament for use in the treatment of, e.g., skin cancer (e.g., melanoma) or a tauopathy (e.g., Alzheimer's disease).

Also described herein is a method of treatment of, e.g., skin cancer (e.g., melanoma) or a tauopathy (e.g., Alzheimer's disease) in a patient, comprising administering to said patient a therapeutically-effective amount of a [¹¹C]-radiolabelled phenothiazine or phenothiazine-like compound as described herein.

Also described herein is a [¹¹C]-radiolabelled phenothiazine or phenothiazine-like compound as described herein for use in a diagnostic or prognostic method practiced on the human or animal body.

Also described herein is a method of diagnosis or prognosis (e.g., of skin cancer (e.g., melanoma) or a tauopathy (e.g., Alzheimer's disease)) which employs a [¹¹C]-radiolabelled phenothiazine or phenothiazine-like compound as described herein.

Also described herein is use of a [¹¹C]-radiolabelled phenothiazine or phenothiazine-like compound as described herein in the manufacture of a medicament (e.g., a diagnostic or prognostic reagent) for use in diagnosis or prognosis, e.g., of skin cancer (e.g., melanoma) or a tauopathy (e.g., Alzheimer's disease).

Also described herein is use of a [¹¹C]-radiolabelled phenothiazine or phenothiazine-like compound as described herein, as part of a method of manufacturing a medicament (e.g., a diagnostic or prognostic reagent) for use in diagnosis or prognosis e.g., of skin cancer (e.g., melanoma) or a tauopathy (e.g., Alzheimer's disease).

Another aspect of the invention pertains to use of:
(i) a phenothiazine compound or a phenothiazine-like compound, wherein:
   said compound has a polycyclic core of three six-membered rings fused together in a linear fashion and denoted the A-ring, B-ring, and C-ring, where the B-ring is the "middle" ring;
   said polycyclic core is partially-aromatic or fully-aromatic;
   said polycyclic core has 14 ring atoms, including exactly 1 or exactly 2 ring heteroatom(s), each of which is independently selected from N, O, and S;
   the remainder of said ring atoms being C;
   said exactly 1 or exactly 2 ring heteroatom(s) form part of the B-ring, but not part of the A-ring or C-ring, and so are located at one or both of the "central" positions denoted by a hash-mark (#) in the following depiction of the polycyclic core: 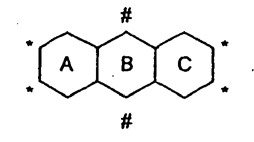
   said compound has a pendant group covalently attached to a ring atom of said polycyclic core;
   said pendant group is independently:
      a primary amino group;
      a cationic primary imino group;
      a secondary amino group;
      a cationic secondary imino group;
      a primary imino group; or
      a secondary imino group;
      and
(ii) [¹¹C]methyl trifluoromethanesulfonate (CF₃SO₂O¹¹CH₃);
   in the manufacture of a medicament (e.g., a diagnostic or prognostic reagent) for use in diagnosis or prognosis, e.g., of skin cancer (e.g., melanoma) or a tauopathy (e.g., Alzheimer's disease).

As will be appreciated by one of skill in the art, features and preferred embodiments of one aspect of the invention will also pertain to other aspects of the invention.

### BRIEF DESCRIPTION OF THE DRAWINGS

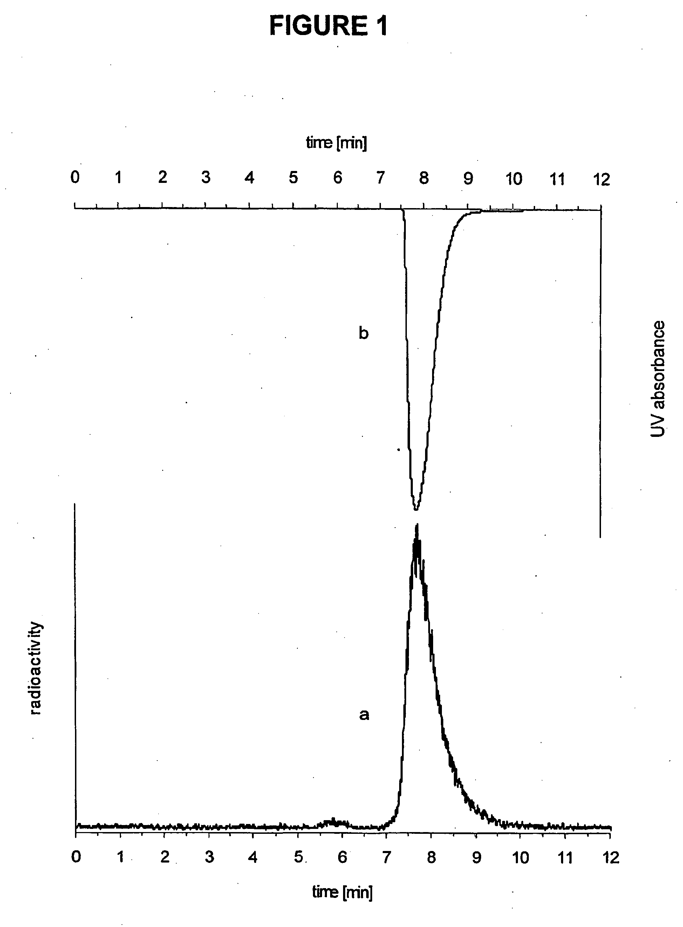

Figure 1 is (a) a radioactivity-chromatogram of [N-methyl-¹¹C]methylene blue (98%, 7.8 minutes) (the minor peak at 5.8 minutes is unidentified) and (b) a UV-chromatogram of non radioactive methylene blue (7.8 minutes).

### DETAILED DESCRIPTION OF THE INVENTION

One aspect of the present invention pertains to methods of [¹¹C]-radiolabelling "phenothiazine" and "phenothiazine-like" compounds, which have a pendant group which is independently:
a primary amino group;
a cationic primary imino group;
a secondary amino group;
a cationic secondary imino group;
a primary imino group; or
a secondary imino group;
by reaction with [¹¹C]methyl trifluoromethanesulfonate (CF₃SO₂O¹¹CH₃), also known as [¹¹C]methyl triflate. This reaction (i.e., ¹¹C-methylation) converts the pendant group into a corresponding [¹¹C]methyl-labelled pendant group, respectively:
a [¹¹C]methyl-labelled secondary amino group;
a [¹¹C]methyl-labelled cationic secondary imino group;
a [¹¹C]methyl-labelled tertiary amino group;
a [¹¹C]methyl-labelled cationic tertiary imino group;
a [¹¹C]methyl-labelled secondary imino group; or
a [¹¹C]methyl-labelled cationic tertiary imino group.

An especially preferred embodiment of novel methods of the present invention is a method of [¹¹C]-radiolabelling Azure B (a "phenothiazine" compound having a pendant secondary amino group; an example of an (unlabelled) phenothiazine compound or (unlabelled) phenothiazine-like compound) to produce [N-methyl-¹¹C]methylene blue, by reaction with the [¹¹C]methyl trifluoromethanesulfonate. In a further preferred embodiment, the reaction is performed in the presence of K₂CO₃ in H₂O, as shown, for example, in the following scheme.

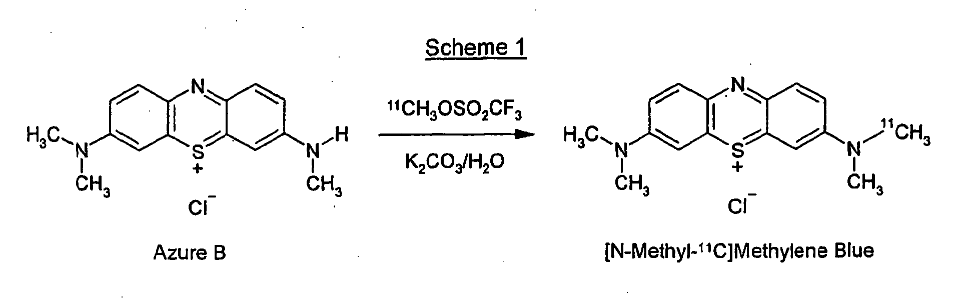

### Reagents, Reaction Conditions, and Purification

In one embodiment, the reaction is performed in the presence of a suitable Bronsted base. Examples of suitable Bronsted bases include, but are not limited to carbonates and bicarbonates, e.g., alkali metal carbonates and bicarbonate, e.g., sodium and potassium carbonates and bicarbonate, e.g., potassium carbonate (K₂CO₃).

In one embodiment, the reaction is carried out in aqueous media. For example, in one embodiment, the [¹¹C]methyl triflate is introduced into an aqueous solution (or suspension) of the phenothiazine or phenothiazine-like compound and (optionally) a suitable Bronsted base, e.g., potassium carbonate (K₂CO₃), to form a reaction mixture.

In one embodiment, the reaction mixture (of [¹¹C]methyl triflate; phenothiazine or phenothiazine-like compound; and optionally Bronsted base) is mixed (e.g., stirred), e.g., for a mixing (e.g., stirring) time of about 1-30 minutes (e.g., about 1-10 minutes; e.g., about 5 minutes).

In one embodiment, the reaction is carried out at ambient or room temperature (e.g., 20°C-25°C).

In one embodiment, the reaction is carried out under an inert atmosphere (e.g., argon).

For example, an argon filled vial equipped with a magnetic stirring bar is filled with a solution of phenothiazine or phenothiazine-like compound and K₂CO₃ in sterile water and subsequently placed on a magnetic stirrer 5 minutes prior to end of bombardment (EOB). [¹¹C]methyl triflate is then trapped in the purple solution. The trapped amount usually reaches a maximum (on average 2.6 GBq) after 15 minutes (from EOB). The magnetic stirrer is then switched on and the solution stirred for 5 minutes at room temperature (e.g., 20°C-25°C) resulting in the [¹¹C]methylation of the phenothiazine or phenothiazine-like compound with [¹¹C]methyl triflate.

In one embodiment, the resulting [¹¹C]-radiolabelled product is purified using ion exchange methods, e.g., with ion exchange media, e.g., using cation exchange methods, e.g., with cation exchange media, e.g., a cation exchange cartridge, e.g., a small disposable cation exchange cartridge.

For example, the reaction mixture may be transferred to a cation exchange cartridge (immobilising the [¹¹C]-radiolabelled product), which is then washed, e.g., with ethanol and sterile water. Washing removes not only unreacted starting material but also up to 98% of the radioactive [¹¹C]by-products. The cartridge is then eluted, e.g., with sodium chloride solution, e.g., sterile 0.9% w/v sodium chloride solution, to release the [¹¹C]-radiolabelled product.

The synthesis (and optionally purification) may readily be performed very quickly, e.g., in less than 60 minutes, e.g., in less than 45 minutes, e.g., in less than 40 minutes, e.g., in less than 35 minutes, e.g., in 10-60 minutes, e.g., in 10-45 minutes, e.g., in 10-40 minutes, e.g., in 10-35 minutes, e.g., in 15-60 minutes, e.g., in 15-45 minutes, e.g., in 15-40 minutes, e.g., in 15-35 minutes, e.g., in 20-60 minutes, e.g., in 20-45 minutes, e.g., in 20-40 minutes, e.g., in 20-35 minutes; from the end of bombardment (EOB).

It is anticipated that synthesis yield and product purity can be further improved by optimisation, for example, optimisation of the bombard time and intensity, reaction solvents, reaction conditions (e.g., temperature), etc.

Radiochemical purity and specific activity of the [¹¹C]-radiolabelled product (solution) may be determined using, for example, HPLC.

The identity of the [¹¹C]-radiolabelled product may be confirmed, for example, by co-injection with the corresponding unlabelled product, and noting that the retention time is identical for both.

In one embodiment, the method provides a radiochemical purity greater than 90%, preferably greater than 95%, preferably greater than 96%, preferably greater than 97%.

In one embodiment, the method provides a radiochemical yield of at least 2%, preferably at least 3%, preferably at least 4%, e.g., 4-10%, e.g., 4-6%.

In one embodiment, the method provides a product with a specific average activity of at least 0.5 GBq/µmol, preferably at least 1.0 GBq/µmol, preferably at least 1.5 GBq/µmol.

### Phenothiazine and Phenothiazine-Like Compounds

The present invention pertains to methods of [¹¹C]-radiolabelling "phenothiazine" and "phenothiazine-like" compounds.

Such compounds are characterized by a polycyclic core of three six-membered rings fused together in a linear fashion, said polycyclic core having 14 ring atoms, including exactly 1 or exactly 2 ring heteroatom(s), each of which is independently selected from nitrogen, oxygen, and sulfur; and remainder of the ring atoms being C. More specifically, one of the ring atoms is independently N, O, or S; another of the ring atoms is independently C, N, O, or S; and the remainder of the ring atoms is C. No other rings are fused to the polycyclic core.

In one embodiment, said polycyclic core has 14 ring atoms, including exactly 1 ring heteroatom selected from nitrogen, oxygen, and sulfur; and the remainder of the rings atoms is C. More specifically, one of the ring atoms is independently N, O, or S; and the remainder of the ring atoms is C.

In one embodiment, said polycyclic core has 14 ring atoms, including exactly 2 ring heteroatoms selected from nitrogen, oxygen, and sulfur; and the remainder of the rings atoms is C. More specifically, one of the ring atoms is independently N, O, or S; another of the ring atoms is independently N, O, or S; and the remainder of the ring atoms is C.

The three six-membered rings are fused together in a linear fashion, and denoted the A-ring, B-ring, and C-ring, where the B-ring is the "middle" ring, as shown in the following depiction of the polycyclic core. 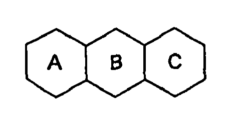 The exactly 1 or exactly 2 ring heteroatom(s) form part of the B-ring, but not part of the A-ring or C-ring, and so are located at one or both of the "central" positions denoted by a hash-mark (#) in the following depiction of the polycyclic core.

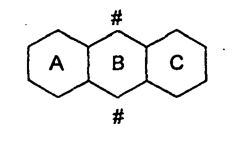

In one embodiment, the core has exactly 1 ring heteroatom.
In one embodiment, the core has exactly 1 ring heteroatom which is independently selected from O, N, and S.
In one embodiment, the core has exactly 1 ring heteroatom which is independently selected from O and N.
In one embodiment, the core has exactly 1 ring heteroatom: O.
In one embodiment, the core has exactly 1 ring heteroatom: N.
In one embodiment, the core has exactly 1 ring heteroatom: S.

In one embodiment, the core has exactly 2 ring heteroatoms.
In one embodiment, the core has exactly 2 ring heteroatoms, each of which is independently selected from O, N and S.
In one embodiment, the core has exactly 2 ring heteroatoms, each of which is independently selected from N and S.
In one embodiment, the core has exactly 2 ring heteroatoms: N and S.
In one embodiment, the core has exactly 2 ring heteroatoms: N and O.
In one embodiment, the core has exactly 2 ring heteroatoms: N and N.
In one embodiment, the core has exactly 2 ring heteroatoms: O and O.
In one embodiment, the core has exactly 2 ring heteroatoms: O and S.
In one embodiment, the core has exactly 2 ring heteroatoms: S and S.

The polycyclic core is partially-aromatic (i.e., not all of the ring atoms contribute to the aromatic character of the polycyclic core), or fully-aromatic (i.e., all of the ring atoms contribute to the aromatic character of the polycyclic core). In one embodiment, the polycyclic core is fully-aromatic.

In one especially preferred embodiment, the exactly 1 or 2 heteroatom(s) are N and S (and are referred to herein as "phenothiazine" compounds):

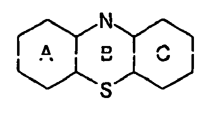

An example of such a polycyclic core is found in phenothiazine:

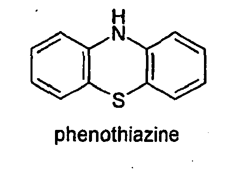

In other embodiments, the exactly 1 or 2 heteroatom(s) are as defined herein, but are other than N and S (and are referred to herein as "phenothiazine-like" compounds).

Examples of such polycyclic cores are found in the following compounds:

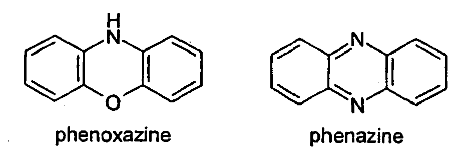

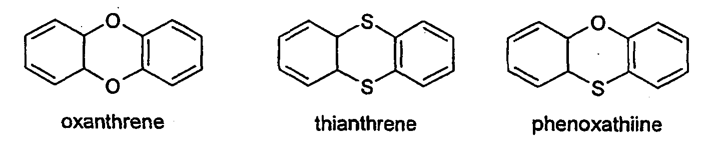

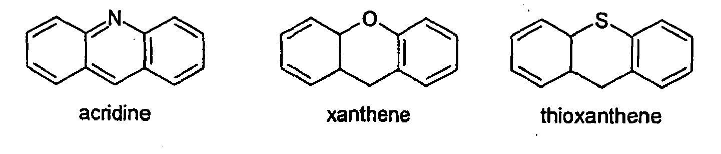

### The Pendant Group

The phenothiazine and phenothiazine-like compounds have a pendant group which is independently:
a primary amino group;
a cationic primary imino group;
a secondary amino group;
a cationic secondary imino group;
a primary imino group; or
a secondary imino group.

The term "pendant group," as used herein, pertains to a group which is covalently attached to a ring atom of the polycyclic core of the phenothiazine compound or phenothiazine-like compound. For example, the pendant group does not form part of a ring of the polycyclic core of (i.e., is not fused to) the phenothiazine compound or phenothiazine-like compound.

A pendant primary amino group is a group of the formula -NH₂.
A pendant cationic primary imino group is =N⁽⁺⁾H₂.

A pendant secondary amino group is a group of the formula -NHR.
A pendant cationic secondary imino group is =N⁽⁺⁾HR.

A pendant primary imino group is a group of the formula =NH.
A pendant secondary imino group is a group of the formula =NR.

Thus, in one embodiment, the pendant group is independently selected from:
-NH₂, -NHR, =N⁽⁺⁾H₂, =N⁽⁺⁾HR, =NH, and =NR.

In one embodiment, the pendant group is independently a secondary amino group or a cationic secondary imino group.

In one embodiment, the pendant group is independently selected from:
-NHR and =N⁽⁺⁾HR.

### The [¹¹C]Methyl Radiolabelled Pendant Group

Upon reaction with the radiolabelled methylating agent, [¹¹C]methyl trifluoromethanesulfonate (CF₃SO₂O¹¹CH₃; [¹¹C]methyl triflate), the pendant group is converted to the corresponding [¹¹C]methyl-labelled pendant group.

Thus, the [¹¹C]methyl-radiolabelled phenothiazine and phenothiazine-like compounds have a pendant group which is independently:
a [¹¹C]methyl-labelled secondary amino group;
a [¹¹C]methyl-labelled cationic secondary imino group;
a [¹¹C]methyl-labelled tertiary amino group;
a [¹¹C]methyl-labelled cationic tertiary imino group;
a [¹¹C]methyl-labelled secondary imino group; or
a [¹¹C]methyl-labelled cationic tertiary imino group.

A pendant primary amino group (-NH₂) gives rise to a corresponding [¹¹C]methyl-labelled secondary amino group: -NH-(¹¹CH₃).

A cationic primary imino group (=N⁽⁺⁾H₂) gives rise to a corresponding [¹¹C]methyl-labelled cationic secondary imino group: =N⁽⁺⁾H-(¹¹CH₃).

A pendant secondary amino group (-NHR) gives rise to a corresponding [¹¹C]methyl-labelled tertiary amino group: -NR-(¹¹CH₃).

A cationic secondary imino group (=N⁽⁺⁾HR) gives rise to a corresponding [¹¹C]methyl-labelled cationic tertiary imino group: =N⁽⁺⁾R-(¹¹CH₃).

A pendant primary imino group (=NH) gives rise to a corresponding [¹¹C]methyl-labelled secondary imino group: =N-(¹¹CH₃).

A pendant secondary imino group (=NR) gives rise to a corresponding [¹¹C]methyl-labelled cationic tertiary imino group: =N⁽⁺⁾R-(¹¹CH₃).

The conversion of the pendant group to the corresponding [¹¹C]methyl-labelled pendant group is summarised in the following table.

| Table 1 | | | |
|---|---|---|---|
| Pendant Group | | Corresponding [¹¹C]Methyl-Labelled Pendant Group | |
| primary amino group | -NH₂ | -NH-(¹¹CH₃) | [¹¹C]methyl-labelled secondary amino group |
| cationic primary imino group | =N⁽⁺⁾H₂ | =N⁽⁺⁾H-(¹¹CH₃) | [¹¹C]methyl-labelled cationic secondary imino group |
| secondary amino group | -NHR | -NR-(¹¹CH₃) | [¹¹C]methyl-labelled tertiary amino group |
| cationic secondary imino group | =N⁽⁺⁾HR | =N⁽⁺⁾R-(¹¹CH₃) | [¹¹C]methyl-labelled cationic tertiary imino group |
| primary imino group | =NH | =N-(¹¹CH₃) | [¹¹C]methyl-labelled secondary imino group |
| secondary imino group | =NR | =N⁽⁺⁾R-(¹¹CH₃) | [¹¹C]methyl-labelled cationic tertiary imino group |

Thus, in one embodiment, the [¹¹C]methyl-labelled pendant group is independently selected from: -NH-(¹¹CH₃), -NR-(¹¹CH₃), =N⁽⁺⁾H-(¹¹CH₃), =N⁽⁺⁾R-(¹¹CH₃), and =N-(¹¹CH₃).

In one embodiment, the [¹¹C]methyl-labelled pendant group is independently a secondary amino group, or a corresponding cationic imino group.

In one embodiment, the [¹¹C]methyl-labelled pendant group is independently selected from: -NR-(¹¹CH₃) and =N⁽⁺⁾R-(¹¹CH₃).

### The Pendant Group: Position

In one embodiment, the pendant group is independently attached to a ring carbon atom of the polycyclic core of the phenothiazine or phenothiazine-like compound.

In one embodiment, the pendant group is independently attached to a ring carbon atom of the A-ring or C-ring of the polycyclic core of the phenothiazine or phenothiazine-like compound.

In one embodiment, the pendant group is independently attached to a ring carbon atom of the A-ring or C-ring, but not of the B-ring, of the polycyclic core of the phenothiazine or phenothiazine-like compound.

In one embodiment, the pendant group is independently attached at one of the "distal" positions of the A-ring or C-ring of the polycyclic core of the phenothiazine or phenothiazine-like compound, which positions are denoted by asterisks (*) in the following depiction of the polycyclic core:

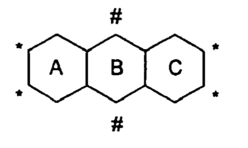

### The Pendant Group: The Substituent R

In one embodiment, R is independently selected from: C₁₋₆alkyl, C₁₋₆alkenyl, C₁₋₆alkynyl, C₁₋₆cycloalkyl, and C₁₋₆cycloalkenyl, and is optionally substituted with one or more (e.g., 1, 2, 3, 4, etc.) groups selected from halo (e.g., fluoro, chloro, bromo, iodo), hydroxy, and C₁₋₄alkoxy.

In one embodiment, R is independently C₁₋₆alkyl.
In one embodiment, R is independently C₁₋₄alkyl.
In one embodiment, R is independently -Me, -Et, -nPr, -iPr, -nBu, -iBu, -sBu, or -tBu.
In one embodiment, R is independently -Me or -Et.
In one embodiment, R is independently -Et.
In one embodiment, R is independently -Me.

### Additional Substituents

In addition to the pendant group discussed above, the phenothiazine or phenothiazine-like compound optionally has one or more (e.g., 1, 2, 3, 4, etc.) additional substituents, for example, selected from: amino (-NH₂), methylamino (-NHMe), dimethylamino (-NMe₂), ethylamino (-NHEt), diethylamino (-NEt₂), imino (=NH), methylimino (=NMe), ethylimino (=NEt), methyl (-Me), ethyl (-Et), fluoro (-F), chloro (-CI), bromo (-Br), iodo (-I), oxo (=O), hydroxy (-OH), carboxy (-COOH), and protonated and deprotonated forms thereof.

### Ionic, Salt, and Solvate Forms

In addition, the phenothiazine or phenothiazine-like compound may be in any ionic (e.g., with a suitable counter-ion), salt (e.g., acid addition salt, e.g., hydrochloride salt), or solvate (e.g., hydrate) form.

For example, an amino group (-NH₂) may be in the form of an HCl addition salt: -NH₂.HCl (or -N⁽⁺⁾H₃Cl⁻).

### Some Preferred Phenothiazine Compounds

In one embodiment, the phenothiazine or phenothiazine-like compound is a compound of the following formula: 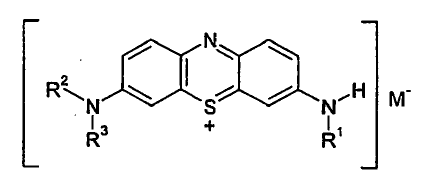 wherein each of R¹, R², and R³ is independently -H or as defined above for R; and M⁻ is an anion (e.g., to achieve electrical neutrality).

In one embodiment, R¹ is independently as defined above for R.

In one embodiment, -NHR¹ is independently -NHMe.

In one embodiment, -NR²R³ is independently -NH₂.
In one embodiment, -NR²R³ is independently -NHMe.
In one embodiment, -NR²R³ is independently -NMe₂.

In one embodiment, M⁻ is independently a halide ion.
In one embodiment, M⁻ is independently F⁻, Cl⁻, Br⁻, or I⁻.
In one embodiment, M⁻ is independently Cl⁻, Br⁻, or I⁻.
In one embodiment, M⁻ is independently Cl⁻.
In one embodiment, M⁻ is independently Br⁻.
In one embodiment, M⁻ is independently I⁻.

In one especially preferred embodiment, phenothiazine or phenothiazine-like compound is Azure B (wherein -NHR¹ is -NHMe; -NR²R³ is -NMe₂; and M⁻ is Cl⁻). 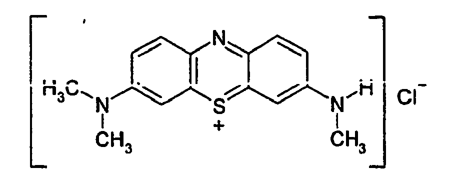 and the resulting [¹¹C]-radiolabelled phenothiazine or phenothiazine-like compounds is [N-methyl-¹¹C]methylene blue:

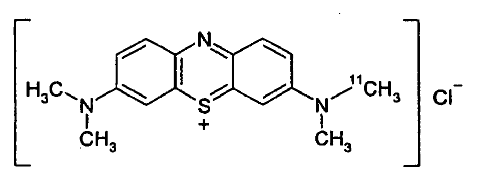

### Resonance Structures

It is noted that may chemical moieties and compounds have resonance properties. Such species may be considered to alternate or resonate between two or more resonance structures. Any of these different resonance structures may be used to accurately represent the species. Usually, but not without exception, the most energetically-stable resonance is used to depict the species. As will be appreciated by the skilled artisan, the structures shown herein are often one of many possible resonance structures which may be drawn to depict the same compound. As used herein, and unless otherwise specified, a reference to one structure is to be considered a reference to all possible corresponding resonance structures.

For example, there are many resonance structures for Azure B, including the ones shown below. Each of these equivalently represents the same compound.

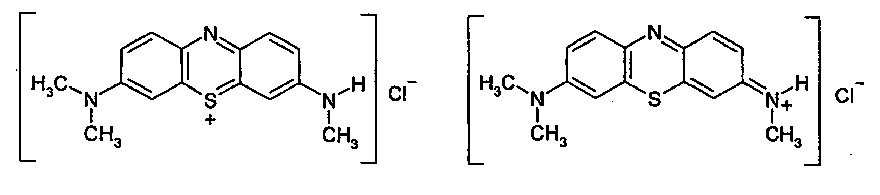

### Some Specific Examples

Some specific examples of phenothiazine and phenothiazine-like compounds include, but are not limited to, the following:

| | |
|---|---|
| 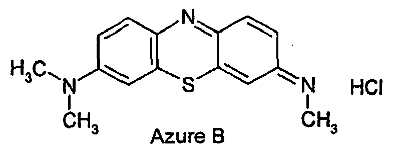 | 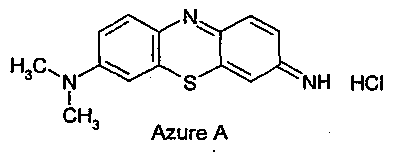 |
| 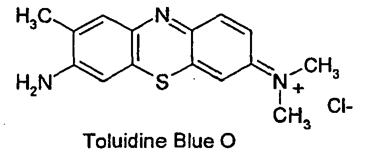 | 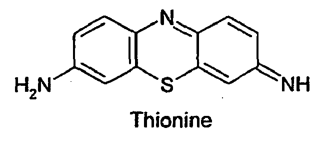 |
| 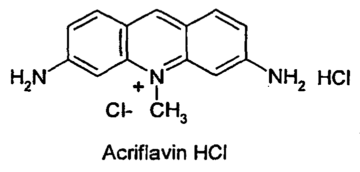 | 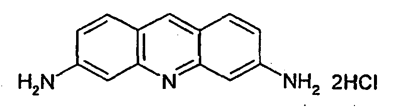 |
| 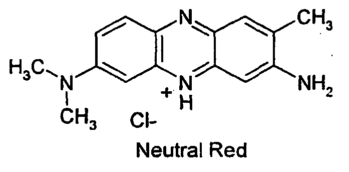 | 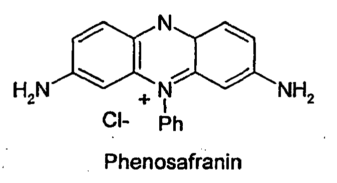 |

### Preparation of Radiolabelled Methylating Reagent: [¹¹C]Methyl Triflate

The methods of the present invention employ the methylating reagent [¹¹C]methyl trifluoromethanesulfonate (CF₃S(=O)₂O-¹¹CH₃), also known as [¹¹C]methyl triflate.

It is noted that [¹¹C]methyl iodide is not only the fastest reacting methyl halide in nucleophilic substitution (S_{N}2) reactions such as N-, O-and S-methylation procedures (see, e.g., Bolton, 2001), but it is also regarded as the most commonly used labelling agent for the preparation of ¹¹C-radiotracers (see, e.g., Nagren et al., 1995). However, efforts to use [¹¹C]methyl iodide as the methylating agent with Azure B have proven unsatisfactory, providing very low radioactive yield and radiochemical purity: the highest radiochemical yield was less than 0.5%.

[¹¹C]Methyl triflate has been used in radioactive labelling reactions (see, e.g., Bolton, 2001; Jewett, 1992; Iwata et al., 2001; Nagren et al., 1995; Lundkvist et al., 1998; Nagren et al., 1998). None of these publications teach or suggest the use of [¹¹C]methyl triflate in the methods described herein.

As demonstrated herein, use of [¹¹C]methyl triflate as a methylating agent greatly increased not only the radioactive yield but also the radiochemical purity.

[¹¹C]Methyl triflate may be prepared, for example, using the methods discussed below.

In a first step ("irradiation"), a mixture of nitrogen and oxygen, at high pressure (e.g., about 1-5 MPa, e.g., about 2 MPa) is subjected to bombardment with high energy (e.g., about 5-20 MeV, e.g., about 10 MeV) protons to form ¹¹CO₂ via a ¹⁴N(p,α)¹¹C nuclear reaction. A beam current of about 10-100 µA (e.g., about 30 µA) and an irradiation time of about 1-120 minutes (e.g., about 10 minutes) is suitable.

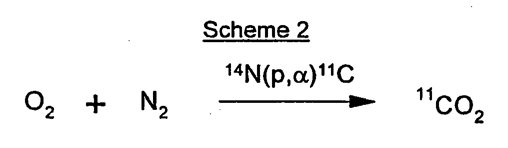

In a second step ("methoxide formation"), the resulting ¹¹CO₂ is reduced to form ¹¹CH₃O⁻, using a suitable reducing agent, for example, lithium aluminium hydride (LiAlH₄, LAH). See, for example, Crouzel et al., 1987. At the "end of bombardment" (EOB), ¹¹CO₂ is transferred, for example, in a stream of helium gas, into a solution of LAH, for example, a cooled 0.1 M solution of LAH in tetrahydrofuran (THF). The ¹¹CO₂ reacts with LAH to produce the ¹¹CH₃O⁻. The solvent (e.g., THF) may be removed by heating, for example, to 130°C.

### Scheme 3

¹¹CO₂ + LiAlH₄ → ¹¹CH₃O⁻ Li⁺

In a third step ("neutralisation"), the resulting ¹¹CH₃O⁻ is neutralised to form the corresponding alcohol, ¹¹CH₃OH, using, for example, a Bronsted acid, for example, phosphoric acid. For example, after removal of solvent, the ¹¹CH₃O⁻ is cooled, for example, to 0°C, phosphoric acid (e.g., 1 ml of 10% phosphoric acid) is added.

### Scheme 4

¹¹CH₃O- Li⁺ + H₃PO₄ → ¹¹CH₃OH + H₂PO₄⁻ Li⁺

In a fourth step ("iodination"), the resulting ¹¹CH₃OH is then reacted with hydroiodic acid (HI). For example, the ¹¹CH₃OH is transferred, e.g., distilled, to another reaction containing HI, and, for example, heated, for example, to 100-150°C (e.g., 135°C) to produce ¹¹CH₃I.

### Scheme 5

¹¹CH₃OH + HI → ¹¹CH₃I + H₂O

In a fifth step ("triflate formation"), the resulting ¹¹CH₃I is then reacted with a suitable triflate salt, for example silver triflate (AgCF₃SO₃). The reaction may conveniently be performed using column methods, for example, using a column packed with silver triflate. See, for example, Jewett, 1992. For example, a suitable column (e.g., stainless steel HPLC C-18 Luna column (250 x 3 mm)) is loosely packed with coarse silver triflate, and held in place with, for example, glass wool. Before use, the column is suitably conditioned, for example, under argon gas flow for 30 minutes at 300°C. The ¹¹CH₃I, in a steam of carrier gas, for example, helium gas, is then passed through the column which is heated to a suitable temperature, for example, about 100-300°C (e.g., about 200°C), to yield the desired CF₃S(=O)₂O-¹¹CH₃.

### Scheme 6

¹¹CH₃I + Ag⁺ CF₃S(=O)₂O⁻ → CF₃S(=O)₂O¹¹CH₃ + Ag⁺ I-

In one embodiment, the methods of [¹¹C]-radiolabelling a phenothiazine compound or a phenothiazine-like compound further comprise the earlier step of (5) triflate formation.

In one embodiment, the methods further comprise the earlier step of (4) iodination and (5) triflate formation.

In one embodiment, the methods further comprise the earlier step of (3) neutralisation, (4) iodination, and (5) triflate formation.

In one embodiment, the methods further comprise the earlier step of (2) methoxide formation, (3) neutralisation, (4) iodination, and (5) triflate formation.

In one embodiment, the methods further comprise the earlier step of (1) irradiation, (2) methoxide formation, (3) neutralisation, (4) iodination, and (5) triflate formation.

### Automation

In one embodiment, the method of [¹¹C]-radiolabelling a phenothiazine compound or a phenothiazine-like compound is partially or fully automated.

In one embodiment, the method is fully automated.

The method may be automated using well known apparatus and techniques.

### [¹¹C]-Radiolabelled Phenothiazine and Phenothiazine-Like Compounds

Also described herein are [¹¹C]-radiolabelled phenothiazine and phenothiazine-like compounds which are obtained by, or are obtainable by, a method as described herein.

Also described herein are [¹¹C]-radiolabelled phenothiazine and phenothiazine-like compound.

In one embodiment, the compound is a [¹¹C]-radiolabelled phenothiazine compound having the following formula wherein R¹, R², R³, and M⁻ is as defined herein:

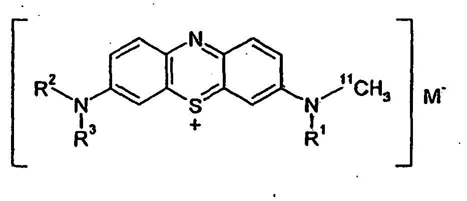

In one embodiment, -NHR¹ is independently -NHMe.
In one embodiment, -NR²R³ is independently -NH₂.
In one embodiment, -NR²R³ is independently -NHMe.
In one embodiment, -NR²R³ is independently -NMe₂.

In one embodiment, M⁻ is independently a halide ion.
In one embodiment, M⁻ is independently Cl⁻.

In an especially preferred embodiment, the compound is [N-methyl-¹¹C]methylene blue:

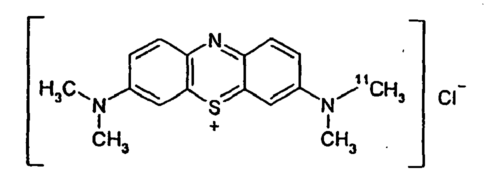

### Compositions

Also described herein are compositions comprising a [¹¹C]-radiolabelled phenothiazine and phenothiazine-like compound, as described herein.

Also described herein are compositions comprising a [¹¹C]-radioiabelled phenothiazine and phenothiazine-like compound which is *obtained by, or is obtainable by,* a method as described herein.

In one embodiment, the composition further comprises a pharmaceutically acceptable carrier.

### Methods of Imaging

Also described herein are methods of (e.g., PET) imaging which employ a [¹¹C]-radiolabelled phenothiazine or phenothiazine-like compound, as described herein.

Also described herein are methods of (e.g., PET) imaging which employ a [¹¹C]-radiolabelled phenothiazine or phenothiazine-like compound which is *obtained by, or is obtainable by,* a method as described herein.

Also described herein are methods of (e.g., PET) imaging which includes, as additional prior steps, the steps of a method of [¹¹C]-radiolabelling a phenothiazine or phenothiazine-like compound, as described herein.

In one embodiment, the methods of imaging comprise the following steps:
(i) introducing the [¹¹C]-radiolabelled phenothiazine or phenothiazine-like compound into a subject;
(ii) imaging (e.g., a part of, the whole of) the subject.

In one embodiment, the step of (ii) imaging the subject is the step of (ii) determining the presence and/or location and/or amount of [¹¹C]-radiolabelled phenothiazine or phenothiazine-like compound in (e.g., a part of, the whole of) the subject.

Methods of PET imaging are well known. See, for example, Czernin et al., 2002; Goh et al., 2003; Van Heertum et al., 2003; Fowler et al., 1999; Kennedy et al., 1997.

For example, in one embodiment, the method is a method of PET imaging comprising the steps of:
(i) preparing a [¹¹C]-radiolabelled phenothiazine or phenothiazine-like compound using a method according to any one of claims 1 to 45;
(ii) introducing said compound into a subject; and
(iii) PET imaging (e.g., a part of, the whole of) the subject.

### Methods of Medical Treatment

Also described herein is a [¹¹C]-radiolabelled phenothiazine or phenothiazine-like compound, as described herein, for use in a method of treatment (e.g., of a disease condition) of the human or animal body by therapy.

Also described herein is a [¹¹C]-radiolabelled phenothiazine or phenothiazine-like compound, which is *obtained by, or is obtainable by,* a method as described herein, for use in a method of treatment (e.g., of a disease condition) of the human or animal body by therapy.

Also described herein is use of a [¹¹C]-radiolabelled phenothiazine or phenothiazine-like compound, as described herein, in the manufacture of a medicament for use in the treatment of a disease condition.

Also described herein is use of a [¹¹C]-radiolabelled phenothiazine or phenothiazine-like compound, which is *obtained by, or is obtainable by,* a method as described herein, in the manufacture of a medicament for use in the treatment of a disease condition.

One aspect of the present invention pertains to use of a method of [¹¹C]-radiolabelling a phenothiazine or a phenothiazine-like compound, as described herein, as part of a method of manufacturing a medicament for use in the treatment of a disease condition.

One aspect of the present invention pertains to a method of manufacturing a medicament for use in the treatment of a disease condition which includes the steps of [¹¹C]-radiolabelling a phenothiazine or a phenothiazine-like compound, as described herein.

One aspect of the present invention pertains to use of:
(i) a (unlabelled) phenothiazine compound or a (unabelled) phenothiazine-like compound, as described herein; and
(ii) [¹¹C]methyl trifluoromethanesulfonate (CF₃SO₂O¹¹CH₃);
in the manufacture of a medicament for use in the treatment of a disease condition.

Also described herein is a method of treatment of a disease condition in a patient, comprising administering to said patient a therapeuticatly-effective amount of a [¹¹C]-radiolabelled phenothiazine or phenothiazine-like compound, as described herein.

Also described herein is a method of treatment of a disease condition in a patient, comprising administering to said patient a therapeutical-effective amount of a [¹¹C]-radiolabelled phenothiazine or phenothiazine-like compound, which is *obtained by, or is obtainable by,* a method as described herein.

Also described herein is a method of treatment of a disease condition in a patient, comprising administering to said patient a therapauticaily-effective amount of a [¹¹C]-radiolabelled phenothiazine or phenothiazine-like compound, and which includes, as additional prior steps, the steps of a method of [¹¹C]-radiolabelling a phenothiazine or phenothiazine-like compound, as described herein.

In one embodiment, the disease condition is skin cancer.
In one embodiment, the disease condition is melanoma.

In one embodiment, the disease condition is a tauopathy.
In one embodiment, the disease condition is Alzheimer's disease (AD).

### Tauopathy

As discussed in Wischik et al., 2002, labelled phenothiazine and phenothiazine-like compounds of the type described herein can bind to "Paired Helical Filaments" (PHFs) and can serve as ligands for tau aggregates.

Such compounds may therefore be used in methods of labelling aggregated PHF tau, for example, for the purpose of diagnosis or prognosis of a tauopathy, such as Alzheimer's Disease (AD).

Notably, it is not only Alzheimer's Disease in which tau protein (and aberrant function or processing thereof) may play a role. The pathogenesis of neurodegenerative disorders such as Pick's disease and Progressive Supranuclear Palsy (PSP) appears to correlate with an accumulation of pathological truncated tau aggregates in the dentate gyrus and stellate pyramidal cells of the neocortex, respectively. Other dementias include fronto-temporal dementia (FTD); parkinsonism linked to chromosome 17 (FTDP-17); disinhibition-dementia-parkinsonism-amyotrophy complex (DDPAC); pallido-ponto-nigral degeneration (PPND); Guam-ALS syndrome; pallido-nigro-luysian degeneration (PNLD); cortico-basal degeneration (CBD) and others (see, e.g., Wischik et al., 2000, especially Table 5.1 therein). Each of these diseases, which is characterized primarily or partially by abnormal tau aggregation, is referred to herein as a "tauopathy."

In particular, the compounds may be used to assess neurofibrillary degeneration associated with tauopathies, e.g., in a subject who may be believed to suffer from any of the above-mentioned diseases.

### Methods of Diagnosis or Prognosis

Also described herein is a [¹¹C]-radiolabelled phenothiazine or phenothiazine-like compound, as described herein; for use in a diagnostic or prognostic method (e.g., of a disease condition) practiced on the human or animal body.

Also described herein is a [¹¹C]-radiolabelled phenothiazine or phenothiazine-like compound, which is *obtained by, or obtainable by,* a method described herein, for use in a diagnostic or prognostic method (e.g., diagnosis or prognosis of a disease condition) practiced on the human or animal body.

Also described herein is a method of diagnosis or prognosis (e.g., of a disease condition) which employs a [¹¹C]-radiolabelled phenothiazine or phenothiazine-like compound, as described herein.

Also described herein is a method of diagnosis or prognosis (e.g., of a disease condition) which employs a [¹¹C]-radiolabelled phenothiazine or phenothiazine-like compound, which is *obtained by, or obtainable by,* a method described herein.

Also described herein is a method of diagnosis or prognosis (e.g., of a disease condition) which employs a [¹¹C]-radiolabelled phenothiazine or phenothiazine-like compound, and which includes, as additional prior steps, the steps of a method of [¹¹C]-radiolabelling a phenothiazine or phenothiazine-like compound, as described herein.

Also described herein is use of a [¹¹C]-radiolabelled phenothiazine or phenothiazine-like compound, as described herein, in the manufacture of a medicament (e.g., a diagnostic or prognostic reagent) for use in the diagnosis or prognosis of a disease condition.

Also described herein is use of a [¹¹C]-radiolabelled phenothiazine or phenothiazine-like compound, which is *obtained by, or obtainable by,* a method described herein, in the manufacture of a medicament (e.g., a diagnostic or prognostic reagent) for use in the diagnosis or prognosis of a disease condition.

One aspect of the present invention pertains to use of a method of [¹¹C]-radiolabelling a phenothiazine or a phenothiazine-like compound, as described herein, as part of a method of preparing a diagnostic or prognostic reagent suitable for use in a method of diagnosis or prognosis (e.g., of a disease condition).

One aspect of the present invention pertains to a method of manufacturing a medicament for use in the diagnosis or prognosis (e.g., of a disease condition) which includes the steps of [¹¹C]-radiolabelling a phenothiazine or a phenothiazine-like compound, as described herein.

One aspect of the present invention pertains to use of:
(i) a (unlabelled) phenothiazine compound or a (unabelled) phenothiazine-like compound, as described herein; and
(ii) [¹¹C]methyl trifluoromethanesulfonate (CF₃SO₂O¹¹CH₃);
in the manufacture of (e.g., in a method of preparing) a medicament (e.g., a diagnostic or prognostic reagent) for use in the diagnosis or prognosis of a disease condition.

In one embodiment, the disease condition is a tauopathy.
In one embodiment, the disease condition is Alzheimer's disease (AD).
In one embodiment, the diagnostic or prognostic method is determining the AD state of a subject.

In one embodiment, the method of diagnosis or prognosis includes, as additional prior steps, the steps of a method of [¹¹C]-radiolabelling a phenothiazine or phenothiazine-like compound, as described herein.

In one embodiment, the methods of diagnosis or prognosis comprise the following steps:
(i) introducing the [¹¹C]-radiolabelled phenothiazine or phenothiazine-like compound into the subject;
(ii) determining the presence and/or location and/or amount of [¹¹C]-radiolabelled phenothiazine or phenothiazine-like compound in the subject;
(iii) correlating the result of the determination made in (ii) with a disease condition of the subject.

In one embodiment, the methods of [¹¹C]-radiolabelling phenothiazine or phenothiazine-like compounds, as described herein, are followed by the additional steps of:
(i) introducing the [¹¹C]-radiolabelled phenothiazine or phenothiazine-like compound into a subject;
(ii) determining the presence and/or location and/or amount of [¹¹C]-radiolabelled phenothiazine or phenothiazine-like compound in the subject;
(iii) correlating the result of the determination made in (ii) with a disease condition of the subject

For example, in one embodiment, the methods of diagnosis or prognosis of a tauopathy comprise the following steps:
(i) introducing the [¹¹C]-radiolabelled phenothiazine or phenothiazine-like compound into the subject;
(ii) determining the presence and/or amount of [¹¹C]-radiolabelled phenothiazine or phenothiazine-like compound bound to aggregated PHF tau in the brain of the subject;
(iii) correlating the result of the determination made in (ii) with the tauopathy (e.g., AD) state of the subject.

In one embodiment, the methods of [¹¹C]-radiolabelling phenothiazine or phenothiazine-like compounds, as described herein, are followed by the additional steps of:
(i) introducing the [¹¹C]-radiolabelled phenothiazine or phenothiazine-like compound into a subject;
(ii) determining the presence and/or amount of [¹¹C]-radiolabelled phenothiazine or phenothiazine-like compound bound to aggregated PHF tau in the brain of the subject;
(iii) correlating the result of the determination made in (ii) with the tauopathy (e.g., AD) state of the subject.

### Treatment

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, regression of the condition, amelioration of the condition, and cure of the condition. Treatment as a prophylactic measure (i.e., prophylaxis, prevention) is also included.

The term "therapeutically-effective amount," as used herein, pertains to that amount of an active compound, or a material, composition or dosage from comprising an active compound, which is effective for producing some desired therapeutic effect, commensurate with a reasonable benefit/risk ratio, when administered in accordance with a desired treatment regimen.

The term "treatment" includes combination treatments and therapies, in which two or more treatments or therapies are combined, for example, sequentially or simultaneously. Examples of treatments and therapies include, but are not limited to, chemotherapy (the administration of active agents, including, e.g., drugs, antibodies (e.g., as in immunotherapy), prodrugs (e.g., as in photodynamic therapy, GDEPT, ADEPT, etc.); surgery; radiation therapy; and gene therapy.

### Routes of Administration

The [¹¹C]-radiolabelled phenothiazine or phenothiazine-like compound, or pharmaceutical composition comprising it, may be administered to a subject/patient by any convenient route of administration, whether systemically/peripherally or topically (i.e., at the site of desired action).

Routes of administration include, but are not limited to, oral (e.g., by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eyedrops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal (including, e.g., intracatheter injection into the brain); by implant of a depot or reservoir, for example, subcutaneously or intramuscularly.

### The Subject/Patient

The subject/patient may be an animal, mammal, a placental mammal, a marsupial (e.g., kangaroo, wombat), a monotreme (e.g., duckbilled platypus), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orangutang, gibbon), or a human.

Furthermore, the subject/patient may be any of its forms of development, for example, a foetus.

In one preferred embodiment, the subject/patient is a human.

### Formulations

While it is possible for the [¹¹C]-radiolabelled phenothiazine or phenothiazine-like compound to be used (e.g., administered) alone, it is often preferable to present it as a formulation.

Also described herein are compositions comprising a [¹¹C]-radiolabelled phenothiazine and phenothiazine-like compound which is obtained by, or is obtainable by, a method as described herein, and a carrier.

Also described herein are compositions comprising a [¹¹C]-radiolabelled phenothiazine and phenothiazine-like compound, as described herein, and a carrier.

In one embodiment, the composition is a pharmaceutical composition (e.g., formulation, preparation, medicament) comprising a compound, as described herein, and a pharmaceutically acceptable carrier.

In one embodiment, the composition is a pharmaceutical composition comprising at least one compound, as described herein, together with one or more other pharmaceutically acceptable ingredients well known to those skilled in the art, including, but not limited to, pharmaceutically acceptable carriers, diluents, excipients, adjuvants, fillers, buffers, preservatives, anti-oxidants, lubricants, stabilisers, solubilisers, surfactants (e.g., wetting agents), masking agents, colouring agents, flavouring agents, and sweetening agents.

In one embodiment, the composition further comprises other active agents, for example, other therapeutic or prophylactic agents.

Suitable carriers, diluents, excipients, etc. can be found in standard pharmaceutical texts. See, for example, Handbook of Pharmaceutical Additives, 2nd Edition (eds. M. Ash and I.

Ash), 2001 (Synapse Information Resources, Inc., Endicott, New York, USA), Remington's Pharmaceutical Sciences, 20th edition, pub. Lippincott, Williams & Wilkins, 2000; and Handbook of Pharmaceutical Excipients, 2nd edition; 1994.

Also described herein are methods of making a pharmaceutical composition comprising admixing at least one [¹¹C]-radiolabelled phenothiazine or phenothiazine-like compound, as defined herein, together with one or more other pharmaceutically acceptable ingredients well known to those skilled in the art, e.g., carriers, diluents, excipients, etc. If formulated as discrete units (e.g., tablets, etc.), each unit contains a predetermined amount (dosage) of the active compound.

The term "pharmaceutically acceptable" as used herein pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, etc. must also be "acceptable" in the sense of being compatible with the other ingredients of the formulation.

The formulations may be prepared by any methods well known in the art of pharmacy. Such methods include the step of bringing into association the active compound with a carrier +which constitutes one or more accessory ingredients. In general, the formulations are prepared by uniformly and intimately bringing into association the active compound with carriers (e.g:, liquid carriers, finely divided solid carrier, etc.), and then shaping the product, if necessary.

The formulation may be prepared to provide for rapid or slow release; immediate, delayed, timed, or sustained release; or a combination thereof.

Formulations suitable for parenteral administration (e.g., by injection), include aqueous or non-aqueous, isotonic, pyrogen-free, sterile liquids (e.g., solutions, suspensions), in which the active ingredient is dissolved, suspended, or otherwise provided (e.g., in a liposome or other microparticulate). Such liquids may additional contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. Typically, the concentration of the active ingredient in the liquid is from about 1 ng/ml to about 10 µg/ml, for example from about 10 ng/ml to about 1 µg/ml. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

### Dosage

It will be appreciated by one of skill in the art that appropriate dosages of the active compounds, and compositions comprising the active compounds, can vary from patient to patient. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition, and the species, sex, age, weight, condition, general health, and prior medical history of the patient. The amount of compound and route of administration will ultimately be at the discretion of the physician, veterinarian, or clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

In general, a suitable dose of the active compound is in the range of about 100 ng to about 25 mg (more typically about 1 µg to about 10 mg) per kilogram body weight of the subject per day. Where the active compound is a salt, an ester, an amide, a prodrug, or the like, the amount administered is calculated on the basis of the parent compound and so the actual weight to be used is increased proportionately.

### EXAMPLES

The following are examples are provided solely to illustrate the present invention and are not intended to limit the scope of the invention, as described herein.

### Chemicals and Solvents

All reagents were purchased from Sigma-Aldrich and used without further purification unless otherwise noted. All used solvents were purified and degassed according to standard procedures.

### Analytical Methods

All analyses of the labelled compounds were performed with a Gynkothek HPLC system (P580 pump) and variable Wavelength UV/VIS detector (at 664 nm) coupled in series with a BIOSCAN Nal detector (B-FC-3200). The HPLC system was operated using a Phenomenex Luna C-18 column (150 x 3.0 mm, particle size: 5 µm). The eluent was produced by adding 0.75% of acetic acid and 0.25% of methane sulfonic acid to a mixture of HPLC grade acetonitrile and distilled water (1:4). The eluent was filtered and degassed with helium before use. The flow rate was set at 1 ml/min.

### Preparation of Silver Trifluoromethanesulfonate Column

A silver trifluoromethanesulfonate (silver triflate) column was prepared according to the method described by Jewett, 1992. Coarse silver triflate (1.0 g) and Graphpac-GC 80/100 (2.0 g, Alltech) was ground to a homogenous mixture. An empty stainless steel HPLC C-18 Luna column (250 x 3 mm) was loosely packed (10 cm length) with the mixture in the central region, and to restrain the packing material, both ends of the column were then fitted with glass wool. Before the first reaction, the column was inserted into a tube furnace (Carbolite furnaces) and conditioned under argon gas flow for 30 minutes at 300°C.

### [¹¹C]Carbon Dioxide Radiosynthesis

[¹¹C]Carbon dioxide was prepared by proton bombardment of a gas mixture (98% N₂, 2% O₂) by the ¹⁴N(p,α)¹¹C nuclear reaction. The gas target was pressurised to 270 psi (1.9 MPa) and irradiated with 11 MeV protons produced by the CTI RDS-111 cyclotron at the John Mallard Scottish P.E.T. Centre in Aberdeen, Scotland. Irradiations of 10 minutes with a beam current of 27 µA were typically used.

### [¹¹C]Methyl Iodide Radiosynthesis

[¹¹C]Methyl iodide was prepared according to the traditional lithium aluminium hydride (LAH)/hydroiodic acid (HI) method (see, for example, Crouzel et al., 1987). At the "end of bombardment" (EOB), [¹¹C]carbon dioxide was transferred from the target in a stream of helium gas to the remote controlled automated [¹¹C]methyl iodide module, where it was passed into 200 µl of a cooled 0.1 M solution of LAH in tetrahydrofuran (THF). The [¹¹C]carbon dioxide reacted with LAH to produce the [¹¹C]methoxide anion. The first reaction vessel was then heated to 130°C to evaporate the solvent. After completing the THF evaporation, the contents of the reaction vessel were cooled to 0°C and 1 ml of 10% phosphoric acid was added to synthesise [¹¹C]methanol. [¹¹C]Methanol was then distilled into the second reaction vessel containing 600 µl of hydroiodic acid (HI). The second reaction vessel was heated to 135°C to produce on average 4.8 GBq of [¹¹C]methyl iodide. The average specific activity was 780 GBq/mmol.

### [¹¹C] Methyl Trifluoromethanesulfonate Radiosynthesis

[¹¹C]Methyl trifluoromethanesulfonate ([¹¹C]methyl triflate) was prepared according to the method described by Jewett, 1992. In a stream of helium gas, the [¹¹C]methyl iodide was passed through the silver triflate graphpac column which was connected in series to the [¹¹C]methyl iodide module. The column was inserted into a tube furnace operated at 200°C, synthesising on average 2.0 GBq of [¹¹C]methyl triflate.

### [N-methyl-¹¹C]Methylene Blue Radiosynthesis

[N-methyl-¹¹C]methylene blue was prepared from Azure B using [¹¹C]methyl triflate. The [¹¹C]methyl triflate was trapped in a reaction vessel containing a solution of Azure B (1 mg, 3.27 µmol) and potassium carbonate (K₂CO₃) (20 mg, 144.72 µmol) in 1.5 mL of sterile water. After the collection of [¹¹C]methyl triflate, the solution was stirred at room temperature (RT, 20°C) for 5 minutes.

The solution was transferred on to a cation exchange cartridge (Waters, Sep-Pak Accell Plus CM) which was washed with 5 ml of ethanol and 15 ml of sterile water. Then the cartridge was eluted with 10 ml of sterile 0.9% w/v sodium chloride solution to yield [N-methyl-[¹¹C]methylene blue. Radiochemical purity and specific activity of the final solution was determined by HPLC.

The identity of the radiolabelled product was confirmed via co-injection with a commercial sample of methylene blue. The retention time in the UV-chromatogram was identical to the retention time of [N-methyl-¹¹C]methylene blue in the radioactivity-chromatogram.

In all cases, [N-methyl-¹¹C]methylene blue was obtained with a radiochemical purity greater than 97% in an averaged 4-6% radiochemical yield based on [¹¹C]methyl iodide. The average specific activity was 1.5G Bq/µmol.

Analytical HPLC showed the product to be >97% radiochemically pure in a 4-6% radiochemical yield and to co-elute with a commercial sample of methylene blue at the same retention time of 7.8 minutes (see Figure 2).

On average, only 7-10 µg/ml of Azure B could be found in the product rinse, as determined by the UV detection spectrum.

The total synthesis time from EOB was 35 minutes.

### REFERENCES

A number of patents and publications are cited above in order to more fully describe and disclose the invention and the state of the art to which the invention pertains. Full citations for these references are provided below.
Bolton R, 2001, "Isotopic methylation," J. Labelled Comp. Radiopharm., Vol. 44, pp. 701-736.
Cancer Research UK Website. http://www.cancerresearchuk.org/aboutcancer/ specific cancers/15216.
Crouzel C, Langstrtim B, Pike VW, Coenen H, 1987, "Recommendations for a practical production of [11C]methyl iodide," Appl. Radiat. Isot., Vol. 38, pp. 601-603.
Czernin J, et al., 2002, "Positron emission tomography scanning: Current and future applications," Annual Review of Medicine, Vol. 53, pp. 89-112.
Fowler JS, et al., 1999, "PET and drug research and development," Journal of Nuclear Medicine, Vol. 40, No. 7, pp. 1154-1163.
Goh ASW et al., 2003, "Clinical positron emission tomography imaging - Current applications," Annals Academy of Medicine Singapore, Vol. 32, No. 4, pp. 507-517.
Iwata R, Pascali C, Bogni A, Miyake Y, Yanai K, Ido T, 2001, "A simple loop method for the automated preparation of [11C]raclopride from [11C]methyl triflate," Appl. Radlat. isot., Vol, 55, pp. 17-22.
Jewett DM, 1992, "A simple synthesis of [11C]methyl triflate," Appl. Rad. Isot., Vol. 43, pp. 1383-1385.
Kennedy SH, et al., 1997, "A review of functional neuroimaging in mood disorders: Positron emission tomography and depression," Canadian Journal of Psychiatry-Revue Canadienne de Psychiatrie, Vol. 42, No. 5, pp. 467-475.
Link EM, Blower PJ, Costa DC, Lane DM, Lui D, Brown RSD, Ell PJ, Spittle MF, 1998, "Early detection of melanoma metastases with radioiodinated methylene blue," Eur. J. Nucl. Med., Vol. 25, pp. 1322-1329.
Lundkvist C, Sandell J, Nagren K, Pike VW, Halldin C, 1998, "Improved synthesis of PET radioligands, [11C]FLB 457, [11C]MDL and [11C]β-CIT-FE, by the use of [11C]methyl triflate," J. Labelled Comp. Radiopharm., Vol. 41, pp. 545-556.
Någren K, Halldin C, 1998, "Methylation of amide and thiol functions with [11C]methyl triflate, as exemplified by [11C]NMSP, [11C]flumazenil and [11C]methionine," J. Labelled Comp. Radiopharm., Vol. 41, pp. 831-841.
Någren K, Müller L, Halldin C, Swahn CG, Lehikoinen P, 1995, "Improved synthesis of some commonly used PET radioligands by the use of [11C]methyl triflate," Nucl. Med. Biol., Vol. 22, pp. 235-239.
Potts AM, 1964, "The reaction of uveal pigment with polycyclic compounds," Invest. Opthmol. Visual. Sci., Vol. 3, pp. 405-416.
Van Heertum RL, et al., 2003, "Positron emission tomography and single-photon emission computed tomography brain imaging in the evaluation of dementia," Seminars in Nuclear Medicine, Vol. 33, No. 1, pp. 77-85.
Wischik et al., 2000, "Neurobiology of Alzheimer's Disease", in The Molecular and Cellular Neurobiology Series, Eds. Dawbarn et al., (Bios Scientific Publishers, Oxford).
Wischik et al., 2002, "Neurofibrillary Labels," published International (PCT) Patent Application publication number WO 02/075318, published 26 September 2002.

## Claims

1. A method of [¹¹C]-radiolabelling a phenothiazine compound or a phenothiazine-like compound, wherein:
said compound has a polycyclic core of three six-membered rings fused together in a linear fashion and denoted the A-ring, B-ring, and C-ring, where the B-ring is the "middle" ring;
said polycyclic core is partially-aromatic or fully-aromatic;
said polycyclic core has 14 ring atoms, including exactly 1 or exactly 2 ring heteroatom(s), each of which is independently selected from N, O, and S;
the remainder of said ring atoms being C;
said exactly 1 or exactly 2 ring heteroatom(s) form part of the B-ring, but not part of the A-ring or C-ring, and so are located at one or both of the "central" positions denoted by a hash-mark (#) in the following depiction of the polycyclic core: 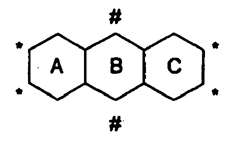
said compound has a pendant group covalently attached to a ring atom of said polycyclic core;
said pendant group is independently:
a primary amino group;
a cationic primary imino group;
a secondary amino group;
a cationic secondary imino group;
a primary imino group; or
a secondary imino group;
said method comprising the step of:
reacting said phenothiazine compound or a phenothiazine-like compound with [¹¹C]methyl trifluoromethanesulfonate (CF₃SO₂O¹¹CH₃);
thereby converting said pendant group to a corresponding [¹¹C]methyl-labelled pendant group, respectively:
a [¹¹C]methyl-labelled secondary amino group;
a [¹¹C]methyl-labelled cationic secondary imino group;
a [¹¹C]methyl-labelled tertiary amino group;
a [¹¹C]methyl-labelled cationic tertiary imino group;
a [¹¹C]methyl-labelled secondary imino group; or
a [¹¹C]methyl-labelled cationic tertiary imino group;
to give a [¹¹C]-radiolabelled phenothiazine or phenothiazine-like compound.

2. A method according to claim 1, wherein said polycyclic core has 14 ring atoms, including exactly 2 ring heteroatoms, each of which is independently selected from N, O, and S.

3. A method according to claim 1, wherein said polycyclic core has 14 ring atoms, including exactly 2 ring heteroatoms: N and S:

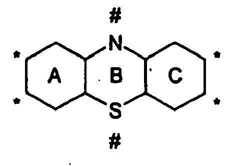

4. A method according to any one of claims 1 to 3, wherein said polycyclic core is fully-aromatic.

5. A method according to any one of claims 1 to 4, wherein said pendant group is independently attached to a ring carbon atom of said polycyclic core.

6. A method according to any one of claims 1 to 4, wherein said pendant group is independently attached to a ring carbon atom of said A-ring or C-ring, but not of said B-ring.

7. A method according to any one of claims 1 to 4, wherein said pendant group is independently attached at one of the "distal" positions of said A-ring or C-ring, which positions are denoted by asterisks (*).

8. A method according to any one of claims 1 to 7, wherein said pendant group is independently:
a secondary amino group or
a cationic secondary imino group;
and said corresponding [¹¹C]methyl-labelled pendant group, respectively, is:
a [¹¹C]methyl-labelled tertiary amino group; or
a [¹¹C]methyl-labelled cationic tertiary imino group.

9. A method according to any one of claims 1 to 7, wherein said pendant group is independently selected from:
-NH₂, -NHR, =N⁽⁺⁾H₂, =N⁽⁺⁾HR, =NH, and =NR;
wherein R is independently selected from C₁₋₆alkyl, C₁₋₆alkenyl, C₁₋₆alkynyl, C₁₋₆cycloalkyl, and C₁₋₆cycloalkenyl, and is optionally substituted with one or more groups selected from fluoro, chloro, bromo, iodo, hydroxy, and C₁₋₄alkoxy;
and said corresponding [¹¹C]methyl-labelled pendant group, respectively, is:
-NH-(¹¹CH₃), -NR-(¹¹CH₃), =N⁽⁺⁾H-(¹¹CH₃), =N⁽⁺⁾R-(¹¹CH₃), or =N-(¹¹CH₃).

10. A method according to any one of claims 1 to 7, wherein said pendant group is independently selected from: -NHR and =N⁽⁺⁾HR;
wherein R is independently selected from C₁₋₆alkyl, C₁₋₆alkenyl, C₁₋₆alkynyl, C₁₋₆cycloalkyl, and C₁₋₆cycloalkenyl, and is optionally substituted with one or more groups selected from fluoro, chloro, bromo, iodo, hydroxy, and C₁₋₄alkoxy;
and said corresponding [¹¹C]methyl-labelled pendant group, respectively, is: -NR-(¹¹CH₃) or =N⁽⁺⁾R-(¹¹CH₃).

11. A method according to claim 9 or 10, wherein R is independently C₁₋₄alkyl.

12. A method according to claim 9 or 10, wherein R is independently -Me or -Et.

13. A method according to claim 9 or 10, wherein R is independently -Me.

14. A method according any one of claims 1 to 13, wherein said compound has, in addition to said pendant group, one or more additional substituents selected from:
amino (-NH₂), methylamino (-NHMe), dimethylamino (-NMe₂), ethylamino (-NHEt), diethylamino (-NEt₂), imino (=NH), methylimino (=NMe), ethylimino (=NEt), methyl (-Me), ethyl (-Et), fluoro (-F), chloro (-Cl), bromo (-Br), iodo (-I), oxo (=O), hydroxy (-OH), carboxy (-COOH), and protonated and deprotonated forms thereof.

15. A method according to claim 1, wherein the phenothiazine or phenothiazine-like compound is a compound of the following formula: 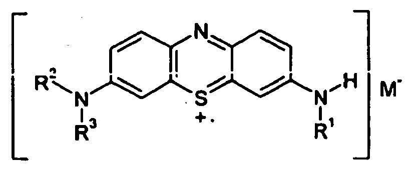 wherein:
each of R¹, R², and R³ is independently -H, C₁₋₆alkyl, C₁₋₆alkenyl, C₁₋₆alkynyl, C₁₋₆cycloalkyl, and C₁₋₆cycloalkenyl, and is optionally substituted with one or more groups selected from fluoro, chloro, bromo, iodo, hydroxy, and C₁₋₄alkoxy; and
M⁻ is an anion.

16. A method according to claim 15, wherein -NHR¹ is independently -NHMe.

17. A method according to claim 15 or 16, wherein -NR²R³ is independently -NH₂.

18. A method according to claim 15 or 16, wherein -NR²R³ is independently -NHMe.

19. A method according to claim 15 or 16, wherein -NR²R³ is independently -NMe₂.

20. A method according to any one of claims 15 to 19, wherein M⁻ is independently a halide ion.

21. A method according to any one of claims 15 to 19, wherein M⁻ is independently Cl⁻.

22. A method according to claim 1, wherein the phenothiazine or phenothiazine-like compound is Azure B: 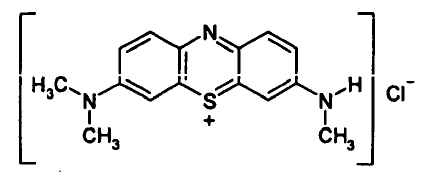 and said [¹¹C]-radiolabelled phenothiazine or phenothiazine-like compound is [N-methyl-¹¹C]methylene blue:

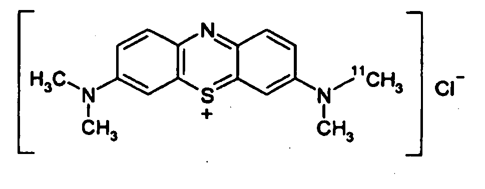

23. A method according to any one of claims 1 to 22, wherein said reaction is performed in the presence of a Bronsted base.

24. A method according to any one of claims 1 to 22, wherein said reaction is performed in the presence of an alkali metal carbonate or bicarbonate.

25. A method according to any one of claims 1 to 22, wherein said reaction is performed in the presence of potassium carbonate (K₂CO₃).

26. A method according to any one of claims 1 to 25, wherein said reaction is carried out in aqueous media.

27. A method according to any one of claims 1 to 25, wherein said reaction is carried out by introducing said [¹¹C]methyl trifluoromethanesulfonate into an aqueous solution or suspension of said phenothiazine or phenothiazine-like compound, to form a reaction mixture.

28. A method according to claim 27, wherein said aqueous solution or suspension further comprises a Bronsted base.

29. A method according to claim 27, wherein said aqueous solution or suspension further comprises an alkali metal carbonate or bicarbonate.

30. A method according to claim 27, wherein said aqueous solution or suspension further comprises potassium carbonate (K₂CO₃).

31. A method according to any one of claims 27 to 30. wherein said reaction mixture is mixed for a mixing time of 1-30 minutes.

32. A method according to any one of claims 27 to 30, wherein said reaction mixture is mixed for a mixing time of 1-10 minutes.

33. A method according to any one of claims 27 to 32, wherein said reaction is carried out at 20°C-25°C.

34. A method according to any one of claims 27 to 32, wherein said reaction is carried out under an inert atmosphere.

35. A method according to any one of claims 27 to 32, wherein said reaction is carried out under argon.

36. A method according to any one of claims 1 to 35, further comprising the subsequent step of:
purifying said [¹¹C]-radiolabelled phenothiazine or phenothiazine-like compound.

37. A method according to any one of claims 1 to 35, further comprising the subsequent step of:
purifying said [¹¹C]-radiolabelled phenothiazine or phenothiazine-like compound using ion exchange methods.

38. A method according to any one of claims 1 to 35, further comprising the subsequent step of:
purifying said [¹¹C]-radiolabelled phenothiazine or phenothiazine-like compound using cation exchange methods.

39. A method according to any one of claims 1 to 38, wherein the reaction and optional purification is performed in less than 60 minutes.

40. A method according to any one of claims 1 to 38, wherein the reaction and optional purification is performed in less than 45 minutes.

41. A method according to any one of claims 1 to 38, wherein the reaction and optional purification is performed in less than 40 minutes.

42. A method according to any one of claims 1 to 41, which provides a radiochemical purity greater than 90%.

43. A method according to any one of claims 1 to 42, which provides a radiochemical yield of at least 2%.

44. A method according to any one of claims 1 to 43, which provides a specific average activity of at least 0.5 GBq/µmol.

45. A method according to any one of claims 1 to 44, which is partially or fully automated.

46. A method of manufacturing a medicament for use in the treatment of skin cancer, melanoma, a tauopathy, or Alzheimer's disease which includes the steps of a method according to any one of claims 1 to 45.

47. A method of manufacturing a medicament for use in the diagnosis or prognosis of skin cancer, melanoma, a tauopathy, or Alzheimer's disease which includes the steps of a method according to any one of claims 1 to 45.

48. Use of:
(i) a phenothiazine compound or a phenothiazine-like compound, wherein:
said compound has a polycyclic core of three six-membered rings fused together in a linear fashion and denoted the A-ring, B-ring, and C-ring, where the B-ring is the "middle" ring;
said polycyclic core is partially-aromatic or fully-aromatic;
said polycyclic core has 14 ring atoms, including exactly 1 or exactly 2 ring heteroatom(s), each of which is independently selected from N, O, and S;
the remainder of said ring atoms being C;
said exactly 1 or exactly 2 ring heteroatom(s) form part of the B-ring, but not part of the A-ring or C-ring, and so are located at one or both of the "central" positions denoted by a hash-mark (#) in the following depiction of the polycyclic core: 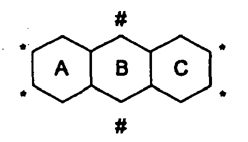
said compound has a pendant group covalently attached to a ring atom of said polycyclic core;
said pendant group is independently:
a primary amino group;
a cationic primary imino group;
a secondary amino group;
a cationic secondary imino group;
a primary imino group; or
a secondary imino group;
and
(ii) [¹¹C]methyl trifluoromethanesulfonate (CF₃SO₂O¹¹CH₃);
in the manufacture of a medicament for use in the treatment of skin cancer, melanoma, a tauopathy, or Alzheimer's disease.

49. Use of:
(i) a phenothiazine compound or a phenothiazine-like compound, wherein:
said compound has a polycyclic core of three six-membered rings fused together in a linear fashion and denoted the A-ring, B-ring, and C-ring, where the B-ring is the "middie" ring;
said polycyclic core is partially-aromatic or fully-aromatic;
said polycyclic core has 14 ring atoms, including exactly 1 or exactly 2 ring heteroatom(s), each of which is independently selected from N, O, and S;
the remainder of said ring atoms being C;
said exactly 1 or exactly 2 ring heteroatom(s) form part of the B-ring, but not part of the A-ring or C-ring, and so are located at one or both of the "central" positions denoted by a hash-mark (#) in the following depiction of the polycyclic core: 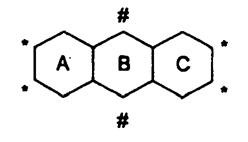
said compound has a pendant group covalently attached to a ring atom of said polycyclic core;
said pendant group is independently:
a primary amino group;
a cationic primary imino group;
a secondary amino group;
a cationic secondary imino group;
a primary imino group; or
a secondary imino group;
and
(ii) [¹¹C]methyl trifluoromethanesulfonate (CF₃SO₂O¹¹CH₃);
in the manufacture of a diagnostic or prognostic reagent for use in the diagnosis or prognosis of skin cancer, melanoma, a tauopathy, or Alzheimer's disease.

## Patentansprüche

1. Verfahren zur radioaktiven Markierung einer Phenothiazinverbindung oder einer phenothiazinartigen Verbindung mit [¹¹C], worin:
die Verbindung einen polyzyklischen Kern aus drei sechsgliedrigen Ringen aufweist, die in linearer Weise aneinander kondensiert sind und als Ring A, Ring B und Ring C bezeichnet werden, worin der Ring B der "mittlere" Ring ist;
der polyzyklische Kern teilweise aromatisch oder vollständig aromatisch ist;
der polyzyklische Kern 14 Ringatome aufweist, einschließlich von genau 1 oder genau 2 Ringheteroatom(en), die jeweils unabhängig voneinander aus N, O und S ausgewählt sind;
der Rest der Ringatome C ist;
das/die genau 1 oder 2 Ringheteroatom(e) einen Teil des Rings B, nicht aber einen Teil des Rings A oder Rings C bildet/-n und sich somit an einer oder beiden der "zentralen" Positionen befindet/-n, die in der folgenden Darstellung des polyzyklischen Kerns mit einer Raute (#) **gekennzeichnet** sind: 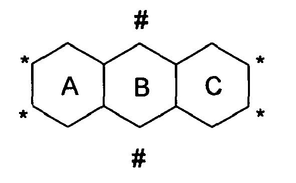
wobei die Verbindung eine Seitengruppe aufweist, die kovalent an ein Ringatom des polyzyklischen Kerns gebunden ist;
wobei die Seitengruppen jeweils unabhängig voneinander:
eine primäre Aminogruppe;
eine kationische primäre Iminogruppe;
eine sekundäre Aminogruppe;
eine kationische sekundäre Iminogruppe;
eine primäre Iminogruppe; oder
eine sekundäre Iminogruppe sind;
wobei das Verfahren folgende Schritte umfasst:
das Umsetzen der Phenothiazinverbindung oder einer phenothiazinartigen Verbindung mit [¹¹C]Methyltrifluormethansulfat (CF₃SO₂O¹¹CH₃);
wodurch die Seitengruppe in eine entsprechende [¹¹C]Methyl-markierte Seitengruppe übergeführt wird bzw. in:
eine [¹¹C]Methyl-markierte sekundäre Aminogruppe;
eine [¹¹C]Methyl-markierte kationische sekundäre Iminogruppe;
eine [¹¹C]Methyl-markierte tertiäre Aminogruppe;
eine [¹¹C]Methyl-markierte kationische tertiäre Iminogruppe;
eine [¹¹C]Methyl-markierte sekundäre Iminogruppe; bzw.
eine [¹¹C]Methyl-markierte kationische tertiäre Iminogruppe;
um eine mit [¹¹C] radioaktiv markierte Phenothiazin- oder phenothiazinartige Verbindung zu erhalten.

2. Verfahren nach Anspruch 1, worin der polyzyklische Kern 14 Ringatome aufweist, einschließlich von genau 2 Ringheteroatomen, die jeweils unabhängig voneinander aus N, O und S ausgewählt sind.

3. Verfahren nach Anspruch 1, worin der polyzyklische Kern 14 Ringatome aufweist, einschließlich von genau 2 Ringheteroatomen: N und S:

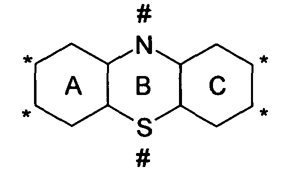

4. Verfahren nach einem der Ansprüche 1 bis 3, worin der polyzyklische Kern vollständig aromatisch ist.

5. Verfahren nach einem der Ansprüche 1 bis 4, worin die Seitengruppen jeweils unabhängig voneinander an einen Ringkohlenstoff des polyzyklischen Kerns gebunden sind.

6. Verfahren nach einem der Ansprüche 1 bis 4, worin die Seitengruppen jeweils unabhängig voneinander an einen Ringkohlenstoff des Rings A oder Rings C, nicht aber des Rings B gebunden sind.

7. Verfahren nach einem der Ansprüche 1 bis 4, worin die Seitengruppen jeweils unabhängig voneinander an eine der mit Sternchen (*) **gekennzeichneten** "distalen" Positionen des Rings A oder Rings C gebunden sind.

8. Verfahren nach einem der Ansprüche 1 bis 7, worin die Seitengruppen jeweils unabhängig voneinander:
eine sekundäre Aminogruppe oder
eine kationische sekundäre Iminogruppe sind;
und die entsprechende [¹¹C]Methyl-markierte Seitengruppe
eine [¹¹C]Methyl-markierte tertiäre Aminogruppe; bzw.
eine [¹¹C]Methyl-markierte kationische tertiäre Iminogruppe ist.

9. Verfahren nach einem der Ansprüche 1 bis 7, worin die Seitengruppen jeweils unabhängig voneinander aus:
- NH₂, -NHR, =N⁽⁺⁾H₂, =N⁽⁺⁾HR, =NH und =Nr. ausgewählt sind;
worin die R jeweils unabhängig voneinander aus C₁₋₆-Alkyl, C₁₋₆-Alkenyl, C₁₋₆-Alkinyl, C₁₋₆-Cycloalkyl und C₁₋₆-Cycloalkenyl ausgewählt sind und gegebenenfalls mit einer oder mehreren aus Fluor, Chlor, Brom, Iod, Hydroxy und C₁₋₄-Alkoxy ausgewählten Gruppen substituiert sind;
und die entsprechende [¹¹C]Methyl-markierte Seitengruppe -NH-(¹¹CH₃), -NR-(¹¹CH₃), =N⁽⁺⁾H-(¹¹CH₃), =N⁽⁺⁾R-(¹¹CH₃) bzw. =N-(¹¹CH₃) ist.

10. Verfahren nach einem der Ansprüche 1 bis 7, worin die Seitengruppen jeweils unabhängig voneinander ausgewählt sind aus: -NHR und =N⁽⁺⁾HR;
worin die R jeweils unabhängig voneinander aus C₁₋₆-Alkyl, C₁₋₆-Alkenyl, C₁₋₆-Alkinyl, C₁₋₆-Cycloalkyl und C₁₋₆-Cycloalkenyl ausgewählt sind und gegebenenfalls mit einer oder mehreren aus Fluor, Chlor, Brom, Iod, Hydroxy und C₁₋₄-Alkoxy ausgewählten Gruppen substituiert sind;
und die entsprechende [¹¹C]Methyl-markierte Seitengruppe -NH-(¹¹CH₃) bzw. =N⁽⁺⁾R-(¹¹CH₃) ist.

11. Verfahren nach Anspruch 9 oder 10, worin die R jeweils unabhängig voneinander C₁₋₄-Alkyl sind.

12. Verfahren nach Anspruch 9 oder 10, worin die R jeweils unabhängig voneinander -Me oder -Et sind.

13. Verfahren nach Anspruch 9 oder 10, worin die R jeweils unabhängig voneinander -Me sind.

14. Verfahren nach einem der Ansprüche 1 bis 13, worin die Verbindung neben der Seitengruppe einen oder mehrere weitere Substituenten aufweist, die ausgewählt sind aus:
Amino (-NH₂), Methylamino (-NHMe), Dimethylamino (-NMe₂), Ethylamino (-NHEt), Diethylamino (-NEt₂), Imino (=NH), Methylimino (=NMe), Ethylimino (=NEt), Methyl (-Me), Ethyl (-Et), Fluor (-F), Chlor (-Cl), Brom (-Br), Iod (-I), Oxo (=O), Hydroxy (-OH), Carboxy (-COOH) sowie protonierten und deprotonierten Formen davon.

15. Verfahren nach Anspruch 1, worin die Phenothiazinverbindung oder phenothiazinartige Verbindung eine Verbindung der folgenden Formel ist: 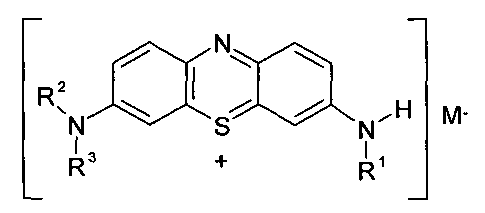 worin:
R¹, R² und R³ jeweils unabhängig voneinander -H, C₁₋₆-Alkyl, C₁₋₆-Alkenyl, C₁₋₆-Alkinyl, C₁₋₆-Cycloalkyl oder C₁₋₆-Cycloalkenyl sind und gegebenenfalls mit einer oder mehreren aus Fluor, Chlor, Brom, Iod, Hydroxy und C₁₋₄-Alkoxy ausgewählten Gruppen substituiert sind; und
M⁻ ein Anion ist.

16. Verfahren nach Anspruch 15, worin die -NHR¹ jeweils unabhängig voneinander -NHMe sind.

17. Verfahren nach Anspruch 15 oder 16, worin die -NR²R³ jeweils unabhängig voneinander -NH₂ sind.

18. Verfahren nach Anspruch 15 oder 16, worin die -NR²R³ jeweils unabhängig voneinander -NHMe sind.

19. Verfahren nach Anspruch 15 oder 16, worin die -NR²R³ jeweils unabhängig voneinander -NMe₂ sind.

20. Verfahren nach einem der Ansprüche 15 bis 19, worin die M⁻ jeweils unabhängig voneinander ein Halogenidion sind.

21. Verfahren nach einem der Ansprüche 15 bis 19, worin die M⁻ jeweils unabhängig voneinander Cl⁻ sind.

22. Verfahren nach Anspruch 1, worin die Phenothiazin- oder phenothiazinartige Verbindung Azur B ist: 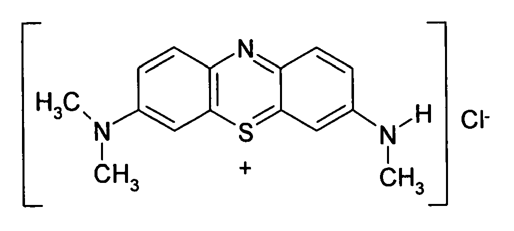 und die mit [¹¹C] radioaktiv markierte Phenothiazin- oder phenothiazinartige Verbindung [N-Methyl-¹¹C]methylenblau ist:

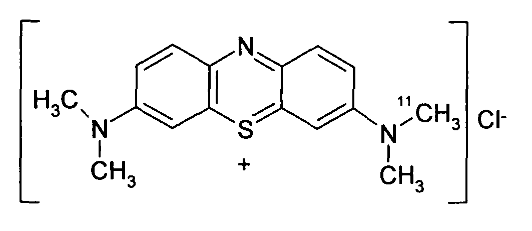

23. Verfahren nach einem der Ansprüche 1 bis 22, worin die Reaktion in Gegenwart einer Brönsted-Base durchgeführt wird.

24. Verfahren nach einem der Ansprüche 1 bis 22, worin die Reaktion in Gegenwart eines Alkalimetallcarbonats oder -bicarbonats durchgeführt wird.

25. Verfahren nach einem der Ansprüche 1 bis 22, worin die Reaktion in Gegenwart von Kaliumcarbonat (K₂CO₃) durchgeführt wird.

26. Verfahren nach einem der Ansprüche 1 bis 25, worin die Reaktion in einem wässrigen Medium durchgeführt wird.

27. Verfahren nach einem der Ansprüche 1 bis 25, worin die Reaktion durch Einführung des [¹¹C]Methyltrifluormethansulfonats in eine wässrige Lösung oder Suspension der Phenothiazin- oder phenothiazinartigen Verbindung zur Bildung eines Reaktionsgemischs durchgeführt wird.

28. Verfahren nach Anspruch 27, worin die wässrige Lösung oder Suspension weiters eine Brönsted-Base umfasst.

29. Verfahren nach Anspruch 27, worin die wässrige Lösung oder Base weiters ein Alkalimetallcarbonat oder -bicarbonat umfasst.

30. Verfahren nach Anspruch 27, worin die wässrige Lösung oder Suspension weiters Kaliumcarbonat (K₂CO₃) umfasst.

31. Verfahren nach einem der Ansprüche 27 bis 30, worin das Reaktionsgemisch eine Mischdauer von 1-30 Minuten lang vermischt wird.

32. Verfahren nach einem der Ansprüche 27 bis 30, worin das Reaktionsgemisch eine Mischdauer von 1-10 Minuten lang vermischt wird.

33. Verfahren nach einem der Ansprüche 27 bis 32, worin die Reaktion bei 20 °C bis 25 °C durchgeführt wird.

34. Verfahren nach einem der Ansprüche 27 bis 32, worin die Reaktion unter einer Inertatmosphäre durchgeführt wird.

35. Verfahren nach einem der Ansprüche 27 bis 32, worin die Reaktion unter Argon durchgeführt wird.

36. Verfahren nach einem der Ansprüche 1 bis 35, die weiters den nachfolgenden, anschließend durchgeführten Schritt umfasst:
Reinigen der mit [¹¹C] radioaktiv markierten Phenothiazin- oder phenothiazinartigen Verbindung.

37. Verfahren nach einem der Ansprüche 1 bis 35, die weiters den nachfolgenden, anschließend durchgeführten Schritt umfasst:
Reinigen der mit [¹¹C] radioaktiv markierten Phenothiazin- oder phenothiazinartigen Verbindung unter Einsatz von Ionenaustauschverfahren.

38. Verfahren nach einem der Ansprüche 1 bis 35, die weiters den nachfolgenden, anschließend durchgeführten Schritt umfasst:
Reinigen der mit [¹¹C] radioaktiv markierten Phenothiazin- oder phenothiazinartigen Verbindung unter Einsatz von Kationenaustauschverfahren.

39. Verfahren nach einem der Ansprüche 1 bis 38, worin die Reaktion und die optionale Reinigung in weniger als 60 Minuten durchgeführt werden.

40. Verfahren nach einem der Ansprüche 1 bis 38, worin die Reaktion und die optionale Reinigung in weniger als 45 Minuten durchgeführt werden.

41. Verfahren nach einem der Ansprüche 1 bis 38, worin die Reaktion und die optionale Reinigung in weniger als 40 Minuten durchgeführt werden.

42. Verfahren nach einem der Ansprüche 1 bis 41, das eine radiochemische Reinheit von über 90 % bereitstellt.

43. Verfahren nach einem der Ansprüche 1 bis 42, das eine radiochemische Ausbeute von zumindest 2 % bereitstellt.

44. Verfahren nach einem der Ansprüche 1 bis 43, das eine mittlere spezifische Aktivität von zumindest 0,5 GBq/µmol bereitstellt.

45. Verfahren nach einem der Ansprüche 1 bis 44, das teilweise oder vollständig automatisiert ist.

46. Verfahren zur Herstellung eines Medikaments zur Verwendung bei der Behandlung von Hautkrebs, Melanomen, einer Tauopathie oder der Alzheimer-Krankheit, welches die Schritte eines Verfahrens nach einem der Ansprüche 1 bis 45 umfasst.

47. Verfahren zur Herstellung eines Medikaments zur Verwendung bei der Diagnose oder Prognose von Hautkrebs, Melanomen, einer Tauopathie oder der Alzheimer-Krankheit, welches die Schritte eines Verfahrens nach einem der Ansprüche 1 bis 45 umfasst.

48. Verwendung von:
(i) einer Phenothiazinverbindung oder einer phenothiazinartigen Verbindung, worin:
die Verbindung einen polyzyklischen Kern aus drei sechsgliedrigen Ringen aufweist, die in linearer Weise aneinander kondensiert sind und als Ring A, Ring B und Ring C bezeichnet werden, worin der Ring B der "mittlere" Ring ist;
der polyzyklische Kern teilweise aromatisch oder vollständig aromatisch ist;
der polyzyklische Kern 14 Ringatome aufweist, einschließlich von genau 1 oder genau 2 Ringheteroatom(en), die jeweils unabhängig voneinander aus N, O und S ausgewählt sind;
der Rest der Ringatome C ist;
das/die genau 1 oder 2 Ringheteroatom(e) einen Teil des Rings B, nicht aber einen Teil des Rings A oder Rings C bildet/-n und sich somit an einer oder beiden der "zentralen" Positionen befindet/-n, die in der folgenden Darstellung des polyzyklischen Kerns mit einer Raute (#) **gekennzeichnet** sind: 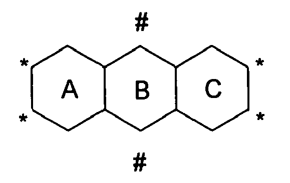
wobei die Verbindung eine Seitengruppe aufweist, die kovalent an ein Ringatom des polyzyklischen Kerns gebunden ist;
wobei die Seitengruppen jeweils unabhängig voneinander:
eine primäre Aminogruppe;
eine kationische primäre Iminogruppe;
eine sekundäre Aminogruppe;
eine kationische sekundäre Iminogruppe;
eine primäre Iminogruppe; oder
eine sekundäre Iminogruppe sind;
und
(ii) [¹¹C]Methyltrifluormethansulfat (CF₃SO₂O¹¹CH₃);
bei der Herstellung eines Medikaments zur Verwendung bei der Behandlung von Hautkrebs, Melanomen, einer Tauopathie oder der Alzheimer-Krankheit.

49. Verwendung von:
(i) einer Phenothiazinverbindung oder einer phenothiazinartigen Verbindung, worin:
die Verbindung einen polyzyklischen Kern aus drei sechsgliedrigen Ringen aufweist, die in linearer Weise aneinander kondensiert sind und als Ring A, Ring B und Ring C bezeichnet werden, worin der Ring B der "mittlere" Ring ist;
der polyzyklische Kern teilweise aromatisch oder vollständig aromatisch ist;
der polyzyklische Kern 14 Ringatome aufweist, einschließlich von genau 1 oder genau 2 Ringheteroatom(en), die jeweils unabhängig voneinander aus N, O und S ausgewählt sind;
der Rest der Ringatome C ist;
das/die genau 1 oder 2 Ringheteroatom(e) einen Teil des Rings B, nicht aber einen Teil des Rings A oder Rings C bildet/-n und sich somit an einer oder beiden der "zentralen" Positionen befindet/-n, die in der folgenden Darstellung des polyzyklischen Kerns mit einer Raute (#) **gekennzeichnet** sind: 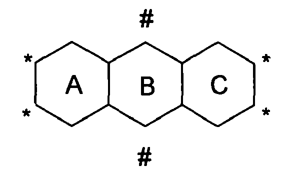
wobei die Verbindung eine Seitengruppe aufweist, die kovalent an ein Ringatom des polyzyklischen Kerns gebunden ist;
wobei die Seitengruppen jeweils unabhängig voneinander:
eine primäre Aminogruppe;
eine kationische primäre Iminogruppe;
eine sekundäre Aminogruppe;
eine kationische sekundäre Iminogruppe;
eine primäre Iminogruppe; oder
eine sekundäre Iminogruppe sind;
und
(ii) [¹¹C]Methyltrifluormethansulfat (CF₃SO₂O¹¹CH₃);
bei der Herstellung eines Medikaments zur Verwendung bei der Diagnose oder Prognose von Hautkrebs, Melanomen, einer Tauopathie oder der Alzheimer-Krankheit.

## Revendications

1. Méthode de radiomarquage par [¹¹C] d'un composé de phénothiazine ou d'un composé ressemblant à la phénothiazine, où
ledit composé a un noyau polycyclique de trois cycles à six membres fusionnés ensemble d'une manière linéaire et désignés par cycle A, cycle B et cycle C, où le cycle B est le cycle "du milieu";
ledit noyau polycyclique est partiellement aromatique ou totalement aromatique;
ledit noyau polycyclique a 14 atomes dans le cycle, comprenant exactement 1 ou exactement 2 hétéroatomes dans le cycle, dont chacun est indépendamment sélectionné parmi N, O et S;
le reste desdits atomes dans le cycle étant C;
lesdits exactement 1 ou exactement 2 hétéroatomes dans le cycle font partie du cycle B, mais non pas partie du cycle A ni du cycle C, et donc ils sont placés à l'une ou aux deux positions "centrales" désignées par un signe dièse (#) dans la représentation suivante du noyau polycyclique: 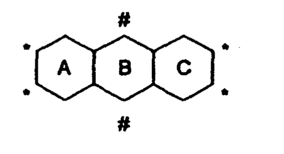 ledit composé a un groupe pendant attaché de manière covalente à un atome du cycle dudit noyau polycyclique;
ledit groupe pendant est indépendamment:
un groupe amino primaire;
un groupe imino primaire cationique;
un groupe amino secondaire;
un groupe imino secondaire cationique;
un groupe imino primaire; ou
un groupe imini secondaire;
ladite méthode comprenant l'étape de:
faire réagir ledit composé de phénothiazine ou un composé ressemblant à la phénothiazine avec du [¹¹C]méthyl trifluorométhanesulfonate (CF₃SO₂O¹¹CH₃);
pour ainsi convertir le groupe pendant en un groupe pendant marqué [¹¹C]méthyle respectivement:
un groupe amino secondaire marqué [¹¹C]méthyle;
un groupe imino secondaire cationique marqué [¹¹C]méthyle;
un groupe amino tertiaire marqué [¹¹C]méthyle;
un groupe imino tertiaire cationique marqué [¹¹C]méthyle;
un groupe imino secondaire marqué [¹¹C]méthyle; ou
un groupe imino tertiaire cationique marqué [¹¹C]méthyle;
pour donner une phénothiazine ou un composé ressemblant à la phénothiazine marqué par [¹¹C].

2. Méthode selon la revendication 1, où ledit noyau polycyclique a 14 atomes dans le cycle, comprenant exactement 2 hétéroatomes dans le cycle, dont chacun est indépendamment sélectionné parmi N, O et S.

3. Méthode selon la revendication 1, où ledit noyau polycyclique a 14 atomes dans le cycle, comprenant exactement 2 hétéroatomes dans le cycle: N et S:

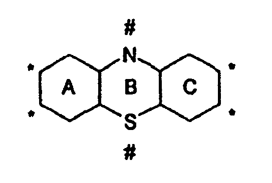

4. Méthode selon l'une quelconque des revendications 1 à 3, où ledit noyau polycyclique est totalement aromatique.

5. Méthode selon l'une quelconques des revendications 1 à 4, où ledit groupe pendant est indépendamment attaché à un atome de carbone du cycle dudit noyau polycyclique.

6. Méthode selon l'une quelconque des revendications 1 à 4, où ledit groupe pendant est indépendamment attaché à un atome de carbone du cycle dudit cycle A ou dudit cycle C, mais non pas dudit cycle B.

7. Méthode selon l'une quelconque des revendications 1 à 4, où ledit groupe est indépendamment attaché à l'une des positions "distales" dudit cycle A ou cycle C, lesquelles positions sont désignées par des astérisques (*) .

8. Méthode selon l'une quelconque des revendications 1 à 7, où ledit groupe pendant est indépendamment:
un groupe amino secondaire ou
un groupe imino secondaire cationique; et ledit groupe pendant marqué [¹¹C]méthyle correspondant respectivement, est:
un groupe amino tertiaire marqué [¹¹C]méthyle; ou
un groupe imino tertiaire cationique marqué [¹¹C]méthyle.

9. Méthode selon l'une quelconque des revendications 1 à 7, où ledit groupe pendant est indépendamment sélectionné parmi:
- NH₂, -NHR, =N⁽⁺⁾H₂, N⁽⁺⁾HR, =NH, et =NR;
où R est indépendamment sélectionné parmi alkyle en C₁₋₆, alcényle en C₁₋₆, alcynyle en C₁₋₆, cycloalkyle en C₁₋₆ et cycloalcényle en C₁₋₆ et est facultativement substitué par un ou plusieurs groupes sélectionnés parmi fluoro, chloro, bromo, iodo, hydroxy et alcoxy en C₁₋₄.
et ledit groupe pendant marqué [¹¹C] méthyle correspondant respectivement est
-NH-(¹¹CH₃), -NR-(¹¹CH₃), =N⁽⁺⁾H-(¹¹H₃), =N⁽⁺⁾R-(¹¹CH₃), ou =N-(¹¹CH₃).

10. Méthode selon l'une quelconque des revendications 1 à 7, où ledit groupe pendant est indépendamment sélectionné parmi: -NHR et =N⁽⁺⁾HR;
où R est indépendamment sélectionné parmi alkyle en C₁₋₆, alcényle en C₁₋₆, alcynyle en C₁₋₆, cycloalkyle en C₁₋₆ et cycloalcényle en C₁₋₆ et est facultativement substitué par un ou plusieurs groupes sélectionnés parmi fluoro, chloro, bromo, iodo, hydroxy et alcoxy en C₁₋₄;
et ledit groupe pendant marqué [¹¹C]méthyle correspondant respectivement est -NR- (¹¹CH₃) ou =N⁽⁺⁾R-(¹¹CH₃).

11. Méthode selon la revendication 9 ou 10, où R est indépendamment alkyle en C₁₋₄.

12. Méthode selon la revendication 9 ou 10, où R est indépendamment -Me ou -Et.

13. Méthode selon la revendication 9 ou 10, où R est indépendamment -Me.

14. Méthode selon l'une quelconque des revendications 1 à 13, où ledit composé a, en plus dudit groupe pendant, un ou plusieurs substituants additionnels sélectionnés parmi:
amino (-NH₂), méthylamino (-NHMe), diméthylamino (-NME₂), éthylamino (-NHEt), diéthylamino (-NEt₂), imino (=NH), méthylimino (=NMe), éthylimino (=NEt), méthyle (-Me), éthyle (-Et), fluoro (-F), chloro (-Cl), bromo (-Br), iodo (-I), oxo (=O), hydroxy (-OH), carboxy (-COOH), et leurs formes protonées et déprotonées.

15. Méthode selon la revendication 1, où la phénothiazine ou le composé ressemblant à la phénothiazine est un composé de la formule suivante: 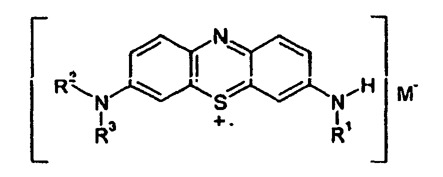 où
chacun de R¹, R² et R³ est indépendamment -H, alkyle en C₁₋₆, alcényle en C₁₋₆, alcynyle en C₁₋₆, cycloalkyle en C₁₋₆ et cycloalcényle en C₁₋₆ et est facultativement substitué par un ou plusieurs groupes sélectionnés parmi fluoro, chloro, bromo, iodo, hydroxy et alcoxy en C₁₋₄; et
M⁻ est un anion.

16. Méthode selon la revendication 15, où -NHR¹ est indépendamment -NHMe.

17. Méthode selon la revendication ou 16, où -NR²R³ est indépendamment -NH₂.

18. Méthode selon la revendication 15 ou 16, où -NR²R³ est indépendamment -NHMe.

19. Méthode selon la revendication 15 ou 16, où -NR²R³ est indépendamment -NMe₂.

20. Méthode selon l'une quelconque des revendications 15 à 19, où M⁻ est indépendamment un ion halogénure.

21. Méthode selon l'une quelconque des revendications 15 à 19, où M⁻ est indépendamment C1⁻.

22. Méthode selon la revendication 1, où la phénothiazine ou le composé ressemblant à la phénothiazine est Azure B: 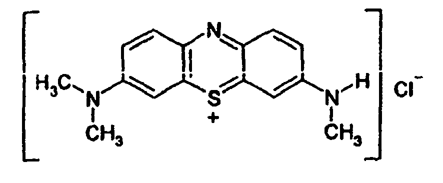 et ladite phénothiazine ou ledit composé ressemblant à la phénothiazine [¹¹C] radiomarqué est le bleu de [N-méthyl-¹¹C]méthylène:

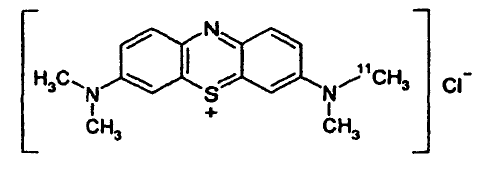

23. Méthode selon l'une quelconque des revendications 1 à 22, où ladite réaction est accomplie en présence d'une base de Bronsted.

24. Méthode selon l'une quelconque des revendications 1 à 22, où ladite réaction est accomplie en présence d'un carbonate ou d'un bicarbonate d'un métal alcalin.

25. Méthode selon l'une quelconque des revendications 1 à 22, où ladite réaction est accomplie en présence de carbonate de potassium (K₂CO₃).

26. Méthode selon l'une quelconque des revendications 1 à 25, où ladite réaction est effectuée dans un milieu aqueux.

27. Méthode selon l'une quelconque des revendications 1 à 25, où ladite réaction est effectuée par introduction dudit [¹¹C]méthyl trifluoro-méthanesulfonate dans une solution aqueuse ou suspension de ladite phénothiazine ou dudit composé ressemblant à la phénothiazine pour former un mélange réactionnel.

28. Méthode selon la revendication 27, où ladite solution ou suspension aqueuse comprend de plus une base de Bronsted.

29. Méthode selon la revendication 27, où ladite solution ou suspension aqueuse comprend de plus un carbonate ou bicarbonate d'un métal alcalin.

30. Méthode selon la revendication 27, où ladite solution ou suspension aqueuse comprend de plus du carbonate de potassium (K₂CO₃).

31. Méthode selon l'une quelconque des revendications 27 à 30, où ledit mélange réactionnel est mélangé pendant une durée de mélange de 1-30 minutes.

32. Méthode selon l'une quelconque des revendications 27 à 30, où ledit mélange réactionnel est mélangé pendant une durée de mélange de 1-10 minutes.

33. Méthode selon l'une des revendications 27 à 32, où ladite réaction est effectuée à 20°C-25°C.

34. Méthode selon l'une quelconque des revendications 27 à 32, où ladite réaction est effectuée sous une atmosphère inerte.

35. Méthode selon l'une quelconque des revendications 27 à 32, où ladite réaction est effectuée sous argon.

36. Méthode selon l'une quelconque des revendications 1 à 35, comprenant de plus l'étape subséquente de:
purifier ladite phénothiazine ou ledit composé ressemblant à la phénothiazine [¹¹C]radiomarqué.

37. Méthode selon l'une quelconque des revendications 1 à 35, comprenant de plus l'étape subséquente de:
purifier ladite phénothiazine ou ledit composé ressemblant à la phénothiazine [¹¹C]radiomarqué en utilisant des méthodes d'échange d'ions.

38. Méthode selon l'une quelconque des revendications 1 à 35, comprenant de plus l'étape subséquente de:
purifier ladite phénothiazine ou ledit composé ressemblant à la phénothiazine [¹¹C]radiomarqué en utilisant des méthodes d'échange de cations.

39. Méthode selon l'une quelconque des revendications 1 à 38, où la rection et la purification facultative est accomplie en moins de 60 minutes.

40. Méthode selon l'une quelconque des revendications 1 à 38, où la réaction et la purification facultative est accomplie en moins de 45 minutes.

41. Méthode selon l'une quelconque des revendications 1 à 38, où la réaction et la purification facultative est accomplie en moins de 40 minutes.

42. Méthode selon l'une quelconque des revendications 1 à 41, qui donne une pureté radiochimique plus importante que 90%.

43. Méthode selon l'une quelconque des revendications 1 à 42, qui donne un rendement radiochimique d'au moins 2%.

44. Méthode selon l'une quelconque des revendications 1 à 43, qui donne une activité moyenne spécifique d'au moins 0,5GBq/µmol.

45. Méthode selon l'une quelconque des revendications 1 à 44, qui est partiellement ou totalement automatisée.

46. Méthode de fabrication d'un médicament à utiliser dans le traitement du cancer de la peau, du mélanome, d'une tauopathie, ou de la maladie d'Alzheimer, qui comprend les étapes d'une méthode selon l'une quelconque des revendications 1 à 45.

47. Méthode de fabrication d'un médicament à utiliser dans le diagnostic ou le pronostic d'un cancer de la peau, d'un mélanome, d'une tauopathie, ou d'une maladie d'Alzheimer, qui comprend les étapes d'une méthode selon l'une quelconque des revendications 1 à 45.

48. Utilisation de:
(i) un composé de phénothiazine ou un composé ressemblant à la phénothiazine, où:
ledit composé a un noyau polycyclique de trois cycles à six membres qui sont fusionnés ensemble d'une manière linéaire et qui sont désignés par cycle A, cycle B et cycle C, où le cycle B est le cycle "du milieu".
ledit noyau polycyclique est partiellement aromatique ou totalement aromatique;
ledit noyau polycyclique a 14 atomes dans le cycle, comprenant exactement 1 ou exactement 2 hétéroatomes dans le cycle, dont chacun est indépendamment sélectionné parmi N, O et S;
le restant desdits atomes dans le cycle étant C;
lesdits exactement 1 ou exactement 2 hétéroatomes dans le cycle font partie du cycle B, mais non pas partie du cycle A ou du cycle C, et ainsi ils sont placés à l'une ou aux deux positions « centrales » désignées par un signe dièse (#) dans la représentation suivante du noyau polycyclique: 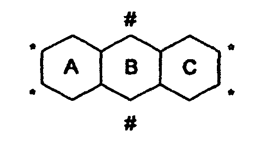
ledit composé a un groupe pendant attaché de manière covalente à un atome du cycle dudit noyau polycyclique;
ledit groupe pendant est indépendamment:
un groupe amino primaire;
un groupe imino primaire cationique;
un groupe amino secondaire;
un groupe imino secondaire cationique;
un groupe imino primaire; ou
un groupe imino secondaire: et
(ii) du [¹¹C]méthyl trifluorométhanesulfonate (CF₃SO₂O¹¹CH₃);
dans la fabrication d'un médicament à utiliser dans le traitement d'un cancer de la peau, d'un mélanome, d'une tauopathie ou de la maladie d'Alzheimer.

49. Utilisation de:
(i) un composé de phénothiazine ou un composé ressemblant à la phénothiazine, où:
ledit composé a un noyau polycyclique de trois cycles à six membres qui sont fusionnés ensemble d'une manière linéaire et qui sont désignés par cycle A, cycle B et cycle C, où le cycle B est le cycle "du milieu".
ledit noyau polycyclique est partiellement aromatique ou totalement aromatique;
ledit noyau polycyclique a 14 atomes dans le cycle, comprenant exactement 1 ou exactement 2 hétéroatomes dans le cycle, dont chacun est indépendamment sélectionné parmi N, O et S;
le restant desdits atomes dans le cycle étant C;
lesdits exactement 1 ou exactement 2 hétéroatomes dans le cycle font partie du cycle B, mais non pas partie du cycle A ou du cycle C, et ainsi ils sont placés à l'une ou aux deux positions "centrales" désignées par un signe dièse (#) dans la représentation suivante du noyau polycyclique: 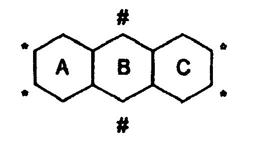
ledit composé a un groupe pendant attaché de manière covalente à un atome du cycle dudit noyau polycyclique;
ledit groupe pendant est indépendamment:
un groupe amino primaire;
un groupe imino primaire cationique;
un groupe amino secondaire;
un groupe imino secondaire cationique;
un groupe imino primaire; ou
un groupe imino secondaire: et
(ii) du [^{11C}]méthyl trifluorométhanesulfonate (CF₃SO₂O¹¹CH₃);
dans la fabrication d'un réactif de diagnostic ou de pronostic à utiliser dans le diagnostic ou le pronostic d'un cancer de la peau, d'un mélanome, d'une tauopathie ou de la maladie d'Alzheimer.
